# EUROPEAN PATENT APPLICATION

(11) **EP 1 612 278 A1**
(43) Date of publication of application: **04.01.2006**
(21) Application number: 05008116.5
(22) Date of filing: 14.04.2005
(51) Int. Cl.: C12Q 1/68

(54) **Method for the detection and identification of circulating micrometastatic cancer cells**

(30) Priority: 02.07.2004 GR 2004100269; 02.07.2004 GR 2004100270; 02.07.2004 GR 2004100271; 02.07.2004 GR 2004100273
(71) Applicant: Acrongenomics Inc., Calgary, Alberta, T2G OT7 (CA)
(72) Inventor: Georgakopoulos, Eleftherios, 15341 Agia Paraskevi Athens (GR)
(74) Representative: Micheli & Cie

(57) **Abstract**

The invention relates to an method for the detection of cancer cells of epithelium origin expressing the Ep-Cam antigen and identification of circulating micrometastatic cancer cells in peripheral blood tissues and other body fluids of patients suffering from cancer of epithelial origin, using a sample of total blood, body fluids or tissue, comprising the amplification of a cDNA molecule encoding the Ep-CAM antigen, which includes the following steps:
(i) hybridizing mRNA isolated by any conventional technique, with a specific (reverse primer) oligonucleotide that hybridizes specifically to the nucleotide sequence of the Ep-CAM antigen;
(ii) producing sequence specific single stranded DNA (complementary DNA) with or without 0.01Mol DTT with the use of reverse transcriptase from mRNA extracted by any conventional technique; and
(iii) detecting Ep-CAM and identifying circulating metastatic cancer cells.

## Description

The present invention relates to a method for the detection of cancer cells of epithelium origin expressing the Ep-Cam antigen and identification of circulating micrometastatic cancer cells in peripheral blood tissues and other body fluids of patients suffering from cancer of epithelial origin, using a sample of total blood, body fluids or tissue.
A characteristic of the malignant tumors is their ability to metastasize. For metastasis to occur, the cancer cells must be able to abandon their attachment to the neighboring cells, to escape from the tissue from which they originate, to penetrate other tissues, until they reach the blood vessels or the vessels of the lymphatic system. There, they must penetrate the basic lamina and enter into the vessels, locate themselves in a certain area of the body where they can survive and reproduce themeselves. The ability of the migrating cancer cells to adhere to the vessels and their penetration is achieved with the expression of integrine that acts as a laminin receptor and the expression of colagen type IV. Every step of these procedures required specific factors. One of them is the expression of Ep-CAM for the cancers of epithelial origin. Being the last development in the cancer treatment the micrometastatic disease and the further developing macrometastatic disease continued to be the major problem for the control of malignancies like colorectal cancer, breast cancer, prostate cancer c.a.
For example, breast cancer patients with metastatic disease have a significantly worse progression-free and overall survival when circulating tumour cells can be detected in their peripheral blood.
The new method is characterized by the detection of cancer micrometastatic cells in peripheral blood of patients with cancer of epithelial origin and is very important for different reasons. These types of cancer represent at least the 80% of human cancers and are very often. The detection of micrometastatic cancer cells in the peripheral blood of patients will help us for the selection of appropriate treatment, would be a clinical tool for the control of the response in the treatment as well as for the control of micrometastasis. Furthermore, with the use of others special markers, it will able to detect the percentage as well as the response of the patient in any treatment schedule.
The development and commercialization of such a diagnostic kit, which is included entire or part of the present invention will constitute the clinical tool for the initiation the treatment, the optimum duration of a therapy, the right dosage and dose, the evaluation of the response and the control of micrometastasis (residual disease).

The main object of the invention is thus a method for the detection of cancer cells of epithelium origin expressing the Ep-Cam antigen and identification of circulating micrometastatic cancer cells in
peripheral blood tissues and other body fluids of patients suffering from cancer of epithelial origin, using a sample of total blood, body fluids or tissue comprising the amplification of a cDNA molecule encoding the Ep-CAM antigen, which includes the following steps:
(i) hybridizing mRNA isolated by any conventional technique, with a specific (reverse primer) oligonucleotide that hybridizes specifically to the nucleotide sequence of the Ep-CAM antigen;
(ii) producing sequence specific single stranded DNA (complementary DNA) with or without 0.01Mol DTT with the use of reverse transcriptase from mRNA extracted by any conventional technique, as follows:
   (a) for each sample preparing a reaction mixture of 10µl end volume comprising :
      - 125µMol of each precursor
      - 10-40U ribonuclease inhibitor
      - 125-250U reverse transcriptase enzyme, and
      - nanoparticles water solution up to final volume of 10 µl;
   (b) adding in a reaction tube :
      - 10-100pMol of reverse primer (sequence ID No2)
      - 1-2µl of total RNA concentrated 1µg/µl, and
      - nanoparticles water solution up to fulfillment of the above volume;
   (c) placing the mixture including the reverse primer on a thermal cycler, and:
      - incubating at 70 °C for 5minutes,
      - placing the tubes on ice,
      - heating the cycler at 37 °C and maintaining this temperature,
      - adding in the tubes that are placed on ice 10µl of the reaction mixture and incubating at :
         37°C, for 10-30min.
         95 °C for 2-5 minutes for the inactivation of the reverse transcriptase enzyme;
(iii) detecting Ep-CAM and identifying circulating micrometastatic cancer cells.

According to particular embodiments of the invention, the detection of Ep-CAM and identification of circulating micrometastatic cancer cells can be carried out either (A) by carrying out PCR reaction, performed under specific conditions with the use of 5µl of the above produced cDNA, nanoparticles water solution, specific pairs of oligonucleotides (primers) that hybridize specifically to the nucleotide sequence of the Ep-CAM antigen mRNA (amplimer forward: 5'- TTA TGA TCC TGA CTG CGA TGA-3' Sequence ID No 1; amplimer reverse: 5'- TTT TCT CTT GCT TTG TGT TTT AGT -3' Sequence ID No 2, where the forward amplimer can be labeled with biotin on one of its end or other) and allows the DNA Taq polymerase to amplify a huge amount of DNA fragments originated from the specific primer pairs used, as follows :
(d) proceeding to the PCR reaction with or without magnesium chloride (MgCl₂) and with or without the reverse primer (amplimer) by adding for each reaction tube and sample :
   - 66.75 µMol of each precursor
   - 50fMol-30pMol of forward primer (sequence ID No1)
   - 50fMol-30pMol of reverse primer (sequence ID No2)
   - 0.6-6U of Taq polymerase, and
   - nanoparticles water solution up to a final volume of 10-30µl;
(e) incubating the above mixture according to a prototype thermical protocol in thermocycler, which is consisting in one of the two following combinations of temperatures and times:
   (1)
      - first annealing step
         94 °C, 24sec-2min.
      - cycling sequence:
         94 °C 6-30sec.
         64 °C 9-45sec.
         72 °C 12-90sec.
         total number of cycles: 8-40
      - final elongation step
         72 °C 2-10min.
         25 °C 4sec.-1min.
   or (2)
      - first annealing step
         94 °C, 30sec - 2min.
      - cycling sequence:
         94 °C 20sec.-80sec.
         53 °C 5sec.- 30sec.
         72 °C 10sec.- 90sec.
         total number of cycles: 9 -35
      - final elongation step
         72 °C 2min.- 7min.
         4 °C until their removal and storage;
(f) conducting the final PCR result analysis;
   or (B) by carrying out Real Time PCR reaction, performed under specific conditions with the use of 5µl of the above produced cDNA, nanoparticles water solution, specific pairs of oligonucleotides (primers) that hybridize specifically to the nucleotide sequence of the Ep-CAM antigen mRNA using a specific probe primer (amplimer forward: 5'- TAA GGC CAA GCA GTG CAA CGG CAC C -3' Sequence ID No 8; amplimer reverse: 5'- TTT TCT CTT GCT TTG TGT TTT AGT -3' Sequence ID No 9; amplimer probe: 5'-FAM TCC AGT AGG TTC TCA CTC GCT CAG A' TAMRA -3' Sequence ID No 10, where the probe can be labeled with fluoroscein on one of its end or other) and allows the DNA Taq polymerase to amplify a huge amount of DNA fragments originated from the specific primer pairs used, as follows :
g) prepare specific cDNA for the gene for Ep-Cam using the cDNA protocol described above with the use of the Real Time PCR specific amplimer reverse (Sequence ID No9);
(h) proceeding to the Real Time PCR reaction with or without magnesium chloride (MgCl₂) and with or without the reverse primer (amplimer) by adding for each reaction tube and sample :
   - 66.75 µMol of each precursor
   - 10pMol-30pMol of forward primer (sequence ID No8)
   - 10pMol-30pMol of reverse primer (sequence ID No9)
   - 10pMol-30pMol of probe primer (sequence ID No10)
   - 1-3U of Taq polymerase, and
   - nanoparticles water solution up to a final volume of 7-10µl;
(i) incubating the above mixture according to a prototype thermical protocol in Real Time PCR thermocycler, which is consisting in the following combinations of temperatures and times:
   - first annealing step
      94 °C, 2sec.
   - cycling sequence:
      94 °C 15sec.
      62 °C 30sec.
      total number of cycles: 13
      10 °C until their removal;
(j) conducting the final Real Time PCR result analysis.

The expression "nanoparticles water solution" which is mentioned in the present specification is to be understood as produced according to conventional protocols, see e.g. Bourlinos et al,. 2003, Microporous and Mesoporous Materials, 58, 155-162; Fournaris et al., 2001, Applied Clay Science 19, 77-88; Bourlinos et al., 2003, 38, 959-963; Bourlinos and Petridis, 2001, 40, 147-151; Voulgaris and Petridis, 2002, 43, 2213-2218; Georgakilas et al., 2004, 42, 865-870.

More particularly, the nanoparticles are in the form of charged spherical particles (of about 0.25 to 500nm) of polystyrene (see Kawasaki et al., J. Material Chemistry 200, 10, 2294-7) and/or of synthetic laponite (aluminositiate mineral), which are coated with two electrolytes, namely poly(dialyldiamethylammonium) chloride, respectively poly(sodium-4-styrene) sulfonate 30% in water.

The coating of the nanoparticles is obtained by using the following methods :
a) To 20ml of white emulsified polystyrene emulsions are added 30ml of a 2% solution of poly (dialyl-diamethylammonium) chloride (SIGMA Aldrich);
   - the mixture is stirred, and then centrifugated at 12,000 rpm during 15 min. at room temperatures;
   - the precipitate obtained is washed with 25ml water, and the washing step is repeated;
   - finally, the precipitate is diluted to 100 ml water.
b) - 728 mg of cetyltrimethylammonium bromide are added to a 1% dispered laponite water solution, and the mixture is stirred, and then centrifugated at 4,000 rpm during 5 min. at room temperature;
   - the solid is washed twice with water and redisperred into 100ml water;
   - 15ml of a 2% solution of poly (sodium-4-styrene)sulfonate are added to the dispersion, and the mixture is stirred during 20min. at room temperature;
   - then the mixture is centrifugated at 12,000 rpm during 15min at room temperature, and the precipitate is washed with 25ml water;
   - the washing step is repeated and the precipitate finally diluted to 100ml water.

According to various embodiments of the invention, the method defined above can also be carried out with the use of different techniques and methodologies for quantification, such as :
i) agarose gel electrophoresis and staining of the PCR products with ethidium bromide, with final visualization under UV radiation and their grabbing;
ii) use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'- GAT CAA TAG TGA TAA CAT TAT TCT C -3'), under the application of any hybridization technique on membrane or in liquids.
iii) marked oligonucleotides (primers) for use in ELISA technique;
iv) marked oligonucleotides (primers) with fluorescent probes to measure the absorbance in fluorometer; or,
v) marked internal oligonucleotide (primers) whose sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'- GAT CAA TAG TGA TAA CAT TAT TCT C-3') for the use in real time PCR or/and in ELISA technique or/and with fluorescent probes to measure the absorbance in fluorometer, and
vi) in case of Real Time PCR detection, the use of nanoparticles water solution, specific pairs of oligonucleotides (primers) that hybridize specifically to the nucleotide sequence of the Ep-CAM antigen mRNA using a specific probe primer (amplimer forward: 5 - TAA GGC CAA GCA GTG CAA CGG CAC C-3' Sequence ID No 8; amplimer reverse: 5'-TTT TCT CTT GCT TTG TGT TTT AGT - 3' Sequence ID No 9; amplimer probe: 5' - FAM TCC AGT AGG TTC TCA CTC GCT CAG A' TAMRA - 3' Sequence ID No 10, where the probe can be labeled with fluoroscein on one of its end or other).

In the case of liquid hybridization the final PCR result analysis will be conducted by adding for each reaction tube and sample:
- 1-15µl PCR product
- 0,2-2pmol of internal probe (Sequence ID 3- Sequence ID 7)
- 100µl nanoparticles water solution

incubating the above mixture in the following combinations of temperatures and times :
2-5min. in 94°C,
5-10min. at room temperature;

transferring the mixture into microplate wells and incubating 5-25min. at 37°C;
washing the microplate wells for 3 times in time intervals of 30sec. with 300µl of PBS-T;
diluting the conjugated antibody in nanoparticles water solution in a rate depending and defined on the using conjugated antibody, and adding to the microplate wells and incubating the above mixture in the following combinations of temperatures and times :
5-25min. at 37°C;
washing the microplate wells for 3 times in time intervals of 30sec. With 300µl of PBS-T;
adding in the microplate wells 100µl of colorimetric substrate in a rate depending and defined on the using conjugated antibody, and incubating for 5-30min.;
and following the addition of 50µl stop solution depending and defined on the using conjugated antibody and colorimetric substrate, detecting the signal in colorimetric plate reader at extension that is dependent and defined on the using conjugated antibody and colorimetric substrate (e.g. 450nm, 405nm, 490nm, 630nm, etc.).

In the case of ELISA, the final PCR result analysis will be conducted by adding for each reaction tube and sample:
- 1-15µl PCR product
- 0,2-2pmol of internal probe (Sequence ID 3- Sequence ID 7)
- 100µl nanoparticles water solution.
incubating the above mixture in the following combinations of temperatures and times:
2-5min. in 94 °C
5-10min. at room temperature;
transferring the mixture into microplate wells and incubating
5-25min. at 37 °C;
washing the microplate wells for 3 times in time intervals of 30sec. with 300µl of PBS-T;
diluting the conjugated antibody in nanoparticles water solution in a rate depending and defined on the using conjugated antibody, and adding to the microplate wells and incubating the above mixture in the following combinations of temperatures and times:
5-25min. at 37 °C;
washing the microplate wells for 3 times in time intervals of 30sec. with 300µl of PBS-T;
adding in the microplate wells 100µl of colorimetric substrate in a rate depending and defined on the using conjugated antibody and will be incubated for 5-30min.; and
following the addition of 50µl stop solution depending and defined on the using conjugated antibody and colorimetric substrate, detecting the signal in colorimetric plate reader at extension that is dependent and defined on the using conjugated antibody and colorimetric substrate (e.g. 450nm, 405nm, 490nm, 630nm, etc.).

Furthermore, for the PCR detection the following compositions can be used :
□ - 66.75 µMol each precursor
   - 3,33 mMol MgCl₂
   - 30pMol forward primer (sequence ID No1)
   - 30pMol reverse primer (sequence ID No2)
   - 0,6U Taq polymerase
   - nanoparticles water solution up to a final volume of 10-30µl.
□ - 66.75 µMol each precursor
   - 30pMol forward primer (sequence ID No1)
   - 30pMol reverse primer (sequence ID No2)
   - 0,6U Taq polymerase
   - nanoparticles water solution up to a final volume of 10-30µl.
□ - 66.75 µMol each precursor
   - 3,33 mMol MgCl₂
   - 10pMol forward primer (sequence ID No1)
   - 10pMol reverse primer (sequence ID No2)
   - 0,6U Taq polymerase
   - nanoparticles water solution up to a final volume of 10-30µl.
□ - 66.75 µMol each precursor
   - 10pMol forward primer (sequence ID No1)
   - 10pMol reverse primer (sequence ID No2)
   - 0,6U Taq polymerase
   - nanoparticles water solution up to a final volume of 10-30µl.
□ - 66.75 µMol each precursor
   - 3,33 mM MgCl₂
   - 5pMol forward primer (sequence ID No1)
   - 5pMol reverse primer (sequence ID No2)
   - 0,6U Taq polymerase
   - nanoparticles water solution up to a final volume of 10-30µl.
□ - 66.75 µMol each precursor
   - 5pMol forward primer (sequence ID No1)
   - 5pMol reverse primer (sequence ID No2)
   - 0,6U Taq polymerase
   - nanoparticles water solution up to a final volume of 10-30µl.
□ - 66.75 µMol each precursor
   - 3,33 mMol MgCl₂
   - 1pMol forward primer (sequence ID No1)
   - 1pMol reverse primer (sequence ID No2)
   - 0,6U Taq polymerase
   - nanoparticles water solution up to a final volume of 10-30µl.
□ - 66.75 µMol each precursor
   - 1pMol forward primer (sequence ID No1)
   - 1pMol reverse primer (sequence ID No2)
   - 0,6U Taq polymerase
   - nanoparticles water solution up to a final volume of 10-30µl.
□ - 66.75 µMol each precursor
   - 100fMol forward primer (sequence ID No1)
   - 100fMol reverse primer (sequence ID No2)
   - 6U Taq polymerase
   - nanoparticles water solution up to a final volume of 10-30µl.
□ - 66.75 µMol each precursor
   - 100fMol forward primer (sequence ID No1)
   - 100fMol reverse primer (sequence ID No2)
   - 3U Taq polymerase
   - nanoparticles water solution up to a final volume of 10-30µl.
□ - 66.75 µMol each precursor
   - 100fMol forward primer (sequence ID No1)
   - 100fMol reverse primer (sequence ID No2)
   - 1,5U Taq polymerase
   - nanoparticles water solution up to a final volume of 10-30µl.
□ - 66.75 µMol each precursor
   - 100fMol forward primer (sequence ID No1)
   - 100fMol reverse primer (sequence ID No2)
   - 0,6U Taq polymerase
   - nanoparticles water solution up to a final volume of 10-30µl.
□ - 66.75 µMol each precursor
   - 50fMo1 forward primer (sequence ID No1)
   - 50fMol reverse primer (sequence ID No2)
   - 6U Taq polymerase
   - nanoparticles water solution up to a final volume of 10-30µl.
□ - 66.75 µMol each precursor
   - 50fMol forward primer (sequence ID No1)
   - 50fMol reverse primer (sequence ID No2)
   - 3U Taq polymerase
   - nanoparticles water solution up to a final volume of 10-30µl.
□ - 66.75 µMol each precursor
   - 50fMol forward primer (sequence ID No1)
   - 50fMol reverse primer (sequence ID No2)
   - 1,5U Taq polymerase
   - nanoparticles water solution up to a final volume of 10-30µl.
□ - 66.75 µMol each precursor
   - 50fMol forward primer (sequence ID No1)
   - 50fMol reverse primer (sequence ID No2)
   - 0,6U Taq polymerase
   - nanoparticles water solution up to a final volume of 10-30µl.
□ - 66.75 µMol each precursor
   - 3,33 mMol MgCl₂
   - 30pMol forward primer (sequence ID No1)
   - 30pMol reverse primer (sequence ID No2)
   - 6U Taq polymerase
   - nanoparticles water solution up to a final volume of 10-30µl.
□ - 66.75 µMol each precursor
   - 3,33 mMol MgCl₂
   - 30pMol forward primer (sequence ID No1)
   - 30pMol reverse primer (sequence ID No2)
   - 3U Taq polymerase
   - nanoparticles water solution up to a final volume of 10-30µl.
□ - 66.75 µMol each precursor
   - 3,33 mMol MgCl₂
   - 30pMol forward primer (sequence ID No1)
   - 30pMol reverse primer (sequence ID No2)
   - 1,5U Taq polymeras
   - nanoparticles water solution up to a final volume of 10-30µl.
□ - 66.75 µMol each precursor
   - 3,33 mMol MgCl₂
   - 10pMol forward primer (sequence ID No1)
   - 10pMol reverse primer (sequence ID No2)
   - 6U Taq polymerase
   - nanoparticles water solution up to a final volume of 10-30µl.
□ - 66.75 µMol each precursor
   - 3,33 mMol MgCl₂
   - 10pMol forward primer (sequence ID No1)
   - 10pMol reverse primer (sequence ID No2)
   - 3U Taq polymerase
   - nanoparticles water solution up to a final volume of 10-30µl.
□ - 66.75 µMol each precursor
   - 3,33 mMol MgCl₂
   - 10pMol forward primer (sequence ID No1)
   - 10pMol reverse primer (sequence ID No2)
   - 1,5U Taq polymerase
   - nanoparticles water solution up to a final volume of 10-30µl.
□ - 66.75 µMol each precursor
   - 3,33 mMol MgCl₂
   - 5pMol forward primer (sequence ID No1)
   - 5pMol reverse primer (sequence ID No2)
   - 6U Taq polymerase
   - nanoparticles water solution up to a final volume of 10-30µl.
□ - 66.75 µMol each precursor
   - 3,33 mMol MgCl₂
   - 5pMol forward primer (sequence ID No1)
   - 5pMol reverse primer (sequence ID No2)
   - 3U Taq polymerase
   - nanoparticles water solution up to a final volume of 10-30µl.
□ - 66.75 µMol each precursor
   - 3,33 mMol MgCl₂
   - 5pMol forward primer (sequence ID No1)
   - 5pMol reverse primer (sequence ID No2)
   - 1,5U Taq polymerase
   - nanoparticles water solution up to a final volume of 10-30µl.
□ - 66.75 µMol each precursor
   - 3,33 mMol MgCl₂
   - 1pMol forward primer (sequence ID No1)
   - 1pMol reverse primer (sequence ID No2)
   - 6U Taq polymerase
   - nanoparticles water solution up to a final volume of 10-30µl.
□ - 66.75 µMol each precursor
   - 3,33 mMol MgCl₂
   - 1pMol forward primer (sequence ID No1)
   - 1pMol reverse primer (sequence ID No2)
   - 3U Taq polymerase
   - nanoparticles water solution up to a final volume of 10-30µl.
□ - 66.75 µMol each precursor
   - 3,33 mMol MgCl₂
   - 1pMol forward primer (sequence ID No1)
   - 1pMol reverse primer (sequence ID No2)
   - 1,5U Taq polymerase
   - nanoparticles water solution up to a final volume of 10-30µl.
□ - 66.75 µMol each precursor
   - 30pMol forward primer (sequence ID No1)
   - 30pMol reverse primer (sequence ID No2)
   - 6U Taq polymerase
   - nanoparticles water solution up to a final volume of 10-30µl.
□ - 66.75 µMol each precursor
   - 30pMol forward primer (sequence ID No1)
   - 30pMol reverse primer (sequence ID No2)
   - 3U Taq polymerase
   - nanoparticles water solution up to a final volume of 10-30µl.
□ - 66.75 µMol each precursor
   - 30pMol forward primer (sequence ID No1)
   - 30pMol reverse primer (sequence ID No2)
   - 1,5U Taq polymerase
   - nanoparticles water solution up to a final volume of 10-30µl.
□ - 66.75 µMol each precursor
   - 10pMol forward primer (sequence ID No1)
   - 10pMol reverse primer (sequence ID No2)
   - 6U Taq polymerase
   - nanoparticles water solution up to a final volume of 10-30µl.
□ - 66.75 µMol each precursor
   - 10pMol forward primer (sequence ID No1)
   - 10pMol reverse primer (sequence ID No2)
   - 3U Taq polymerase
   - nanoparticles water solution up to a final volume of 10-30µl.
□ - 66.75 µMol each precursor
   - 10pMol forward primer (sequence ID No1)
   - 10pMol reverse primer (sequence ID No2)
   - 1,5U Taq polymerase
   - nanoparticles water solution up to a final volume of 10-30µl.
□ - 66.75 µMol each precursor
   - 5pMol forward primer (sequence ID No1)
   - 5pMol reverse primer (sequence ID No2)
   - 6U Taq polymerase
   - nanoparticles water solution up to a final volume of 10-30µl.
□ - 66.75 µMol each precursor
   - 5pMol forward primer (sequence ID No1)
   - 5pMol reverse primer (sequence ID No2)
   - 3U Taq polymerase
   - nanoparticles water solution up to a final volume of 10-30µl.
□ - 66.75 µMol each precursor
   - 5pMol forward primer (sequence ID No1)
   - 5pMol reverse primer (sequence ID No2)
   - 1,5U Taq polymerase
   - nanoparticles water solution up to a final volume of 10-30µl.
□ - 66.75 µMol each precursor
   - 1pMol forward primer (sequence ID No1)
   - 1pMol reverse primer (sequence ID No2)
   - 6U Taq polymerase
   - nanoparticles water solution up to a final volume of 10-30µl.
□ - 66.75 µMol each precursor
   - 1pMol forward primer (sequence ID No1)
   - 1pMol reverse primer (sequence ID No2)
   - 3U Taq polymerase
   - nanoparticles water solution up to a final volume of 10-30µl.
□ - 66.75 µMol each precursor
   - 1pMol forward primer (sequence ID No1)
   - 1pMol reverse primer (sequence ID No2)
   - 1,5U Taq polymerase
   - nanoparticles water solution up to a final volume of 10-30µl.
□ - 66.75 µMol each precursor
   - 30pMol forward primer (sequence ID No1)
   - 6U Taq polymerase
   - nanoparticles water solution up to a final volume of 10-30µl.
□ - 66.75 µMol each precursor
   - 30pMol forward primer (sequence ID No1)
   - 3U Taq polymerase
   - nanoparticles water solution up to a final volume of 10-30µl.
□ - 66.75 µMol each precursor
   - 30pMol forward primer (sequence ID No1)
   - 1,5U Taq polymerase
   - nanoparticles water solution up to a final volume of 10-30µl.
□ - 66.75 µMol each precursor
   - 30pMol forward primer (sequence ID No1)
   - 0,6U Taq polymerase
   - nanoparticles water solution up to a final volume of 10-30µl.
□ - 66.75 µMol each precursor
   - 10pMol forward primer (sequence ID No1)
   - 6U Taq polymerase
   - nanoparticles water solution up to a final volume of 10-30µl.
□ - 66.75 µMol each precursor
   - 10pMol forward primer (sequence ID No1)
   - 3U Taq polymerase
   - nanoparticles water solution up to a final volume of 10-30µl.
□ - 66.75 µMol each precursor
   - 10pMol forward primer (sequence ID No1)
   - 1,5U Taq polymerase
   - nanoparticles water solution up to a final volume of 10-30µl.
□ - 66.75 µMol each precursor
   - 10pMol forward primer (sequence ID No1)
   - 0,6U Taq polymerase
   - nanoparticles water solution up to a final volume of 10-30µl.
□ - 66.75 µMol each precursor
   - 5pMol forward primer (sequence ID No1)
   - 6U Taq polymerase
   - nanoparticles water solution up to a final volume of 10-30µl.
□ - 66.75 µMol each precursor
   - 5pMol forward primer (sequence ID No1)
   - 3U Taq polymerase
   - nanoparticles water solution up to a final volume of 10-30µl.
□ - 66.75 µMol each precursor
   - 5pMol forward primer (sequence ID No1)
   - 1,5U Taq polymerase
   - nanoparticles water solution up to a final volume of 10-30µl.
□ - 66.75 µMol each precursor
   - 5pMol forward primer (sequence ID No1)
   - 0,6U Taq polymerase
   - nanoparticles water solution up to a final volume of 10-30µl.
□ - 66.75 µMol each precursor
   - 1pMol forward primer (sequence ID No1)
   - 6U Taq polymerase
   - nanoparticles water solution up to a final volume of 10-30µl.
□ - 66.75 µMol each precursor
   - 1pMol forward primer (sequence ID No1)
   - 3U Taq polymerase
   - nanoparticles water solution up to a final volume of 10-30µl.
□ - 66.75 µMol each precursor
   - 1pMol forward primer (sequence ID No1)
   - 1,5U Taq polymerase
   - nanoparticles water solution up to a final volume of 10-30µl.
□ - 66.75 µMol each precursor
   - 1pMol forward primer (sequence ID No1)
   - 0,6U Taq polymerase
   - nanoparticles water solution up to a final volume of 10-30µl.
□ - 66.75 µMol each precursor
   - 100fMol forward primer (sequence ID No1)
   - 6U Taq polymerase
   - nanoparticles water solution up to a final volume of 10-30µl.
□ - 66.75 µMol each precursor
   - 100fMol forward primer (sequence ID No1)
   - 3U Taq polymerase
   - nanoparticles water solution up to a final volume of 10-30µl.
□ - 66.75 µMol each precursor
   - 100fMol forward primer (sequence ID No1)
   - 1,5U Taq polymerase
   - nanoparticles water solution up to a final volume of 10-30µl.
□ - 66.75 µMol each precursor
   - 100fMol forward primer (sequence ID No1)
   - 0,6U Taq polymerase
   - nanoparticles water solution up to a final volume of 10-30µl.
□ - 66.75 µMol each precursor
   - 50fMol forward primer (sequence ID No1)
   - 6U Taq polymerase
   - nanoparticles water solution up to a final volume of 10-30µl.
□ - 66.75 µMol each precursor
   - 50fMol forward primer (sequence ID No1)
   - 3U Taq polymerase
   - nanoparticles water solution up to a final volume of 10-30µl.
□ - 66.75 µMol each precursor
   - 50fMol forward primer (sequence ID No1)
   - 1,5U Taq polymerase
   - nanoparticles water solution up to a final volume of 10-30µl., and
□ - 66.75 µMol each precursor
   - 50fMol forward primer (sequence ID No1)
   - 0,6U Taq polymerase
   - nanoparticles water solution up to a final volume of 10-30µl.

Furthermore, for the Real Time PCR detection the following compositions can be used:
□ - 66.75 µMol each precursor
   - 30pMol forward primer (sequence ID No8)
   - 30pMol reverse primer (sequence ID No9)
   - 30pMol probe (sequence ID No10)
   - 3U Taq polymerase
   - nanoparticles water solution up to a final volume of 7-10µl.
□ - 66.75 µMol each precursor
   - 30pMol forward primer (sequence ID No8)
   - 30pMol reverse primer (sequence ID No9)
   - 30pMol probe (sequence ID No10)
   - 1.5 U Taq polymerase
   - nanoparticles water solution up to a final volume of 7-10µl.
□ - 66.75 µMol each precursor
   - 30pMol forward primer (sequence ID No8)
   - 30pMol reverse primer (sequence ID No9)
   - 30pMol probe (sequence ID No10)
   - 1U Taq polymerase
   - nanoparticles water solution up to a final volume of 7-10µl.
□ - 66.75 µMol each precursor
   - 25pMol forward primer (sequence ID No8)
   - 25pMol reverse primer (sequence ID No9)
   - 25pMol probe (sequence ID No10)
   - 3 U Taq polymerase
   - nanoparticles water solution up to a final volume of 7-10µl.
□ - 66.75 µMol each precursor
   - 25pMol forward primer (sequence ID No8)
   - 25pMol reverse primer (sequence ID No9)
   - 25pMol probe (sequence ID No10)
   - 1.5U Taq polymerase
   - nanoparticles water solution up to a final volume of 7-10µl.
□ - 66.75 µMol each precursor
   - 25pMol forward primer (sequence ID No8)
   - 25pMol reverse primer (sequence ID No9)
   - 25pMol probe (sequence ID No10)
   - 1 U Taq polymerase
   - nanoparticles water solution up to a final volume of 7-10µl.
□ - 66.75 µMol each precursor
   - 20pMol forward primer (sequence ID No8)
   - 20pMol reverse primer (sequence ID No9)
   - 20pMol probe (sequence ID No10)
   - 3U Taq polymerase
   - nanoparticles water solution up to a final volume of 7-10µl.
□ - 66.75 µMol each precursor
   - 20pMol forward primer (sequence ID No8)
   - 20pMol reverse primer (sequence ID No9)
   - 20pMol probe (sequence ID No10)
   - 1.5U Taq polymerase
   - nanoparticles water solution up to a final volume of 7-10µl.
□ - 66.75 µMol each precursor
   - 20pMol forward primer (sequence ID No8)
   - 20pMol reverse primer (sequence ID No9)
   - 20pMol probe (sequence ID No10)
   - 1U Taq polymerase
   - nanoparticles water solution up to a final volume of 7-10µl.
□ - 66.75 µMol each precursor
   - 15pMol forward primer (sequence ID No8)
   - 15pMol reverse primer (sequence ID No9)
   - 15pMol probe (sequence ID No10)
   - 3U Taq polymerase
   - nanoparticles water solution up to a final volume of 7-10µl.
□ - 66.75 µMol each precursor
   - 15pMol forward primer (sequence ID No8)
   - 15pMol reverse primer (sequence ID No9)
   - 15pMol probe (sequence ID No10)
   - 1.5U Taq polymerase
   - nanoparticles water solution up to a final volume of 7-10µl.
□ - 66.75 µMol each precursor
   - 15pMol forward primer (sequence ID No8)
   - 15pMol reverse primer (sequence ID No9)
   - 15pMol probe (sequence ID No10)
   - 1U Taq polymerase
   - nanoparticles water solution up to a final volume of 7-10µl.
□ - 66.75 µMol each precursor
   - 10pMol forward primer (sequence ID No8)
   - 10pMol reverse primer (sequence ID No9)
   - 10pMol probe (sequence ID No10)
   - 1.5 U Taq polymerase
   - nanoparticles water solution up to a final volume of 7-10µl.
□ - 66.75 µMol each precursor
   - 10pMol forward primer (sequence ID No8)
   - 10pMol reverse primer (sequence ID No9)
   - 10pMol probe (sequence ID No10)
   - 1 U Taq polymerase
   - nanoparticles water solution up to a final volume of 7-10µl.
□ - 66.75 µMol each precursor
   - 3,33 mMol MgCl₂
   - 30pMol forward primer (sequence ID No8)
   - 30pMol reverse primer (sequence ID No9)
   - 30pMol probe (sequence ID No10)
   - 3U Taq polymerase
   - nanoparticles water solution up to a final volume of 7-10µl.
□ - 66.75 µMol each precursor
   - 3,33 mMol MgCl₂
   - 30pMol forward primer (sequence ID No8)
   - 30pMol reverse primer (sequence ID No9)
   - 30pMol probe (sequence ID No10)
   - 1.5 U Taq polymerase
   - nanoparticles water solution up to a final volume of 7-10µl.
□ - 66.75 µMol each precursor
   - 3,33 mMol MgCl₂
   - 30pMol forward primer (sequence ID No8)
   - 30pMol reverse primer (sequence ID No9)
   - 30pMol probe (sequence ID No10)
   - 1U Taq polymerase
   - nanoparticles water solution up to a final volume of 7-10µl.
□ - 66.75 µMol each precursor
   - 3,33 mMol MgCl₂
   - 25pMol forward primer (sequence ID No8)
   - 25pMol reverse primer (sequence ID No9)
   - 25pMol probe (sequence ID No10)
   - 3 U Taq polymerase
   - nanoparticles water solution up to a final volume of 7-10µl.
□ - 66.75 µMol each precursor
   - 3,33 mMol MgCl₂
   - 25pMol forward primer (sequence ID No8)
   - 25pMol reverse primer (sequence ID No9)
   - 25pMol probe (sequence ID No10)
   - 1.5U Taq polymerase
   - nanoparticles water solution up to a final volume of 7-10µl.
□ - 66.75 µMol each precursor
   - 3,33 mMol MgCl₂
   - 25pMol forward primer (sequence ID No8)
   - 25pMol reverse primer (sequence ID No9)
   - 25pMol probe (sequence ID No10)
   - 1 U Taq polymerase
   - nanoparticles water solution up to a final volume of 7-10µl.
□ - 66.75 µMol each precursor
   - 3,33 mMol MgCl₂
   - 20pMol forward primer (sequence ID No8)
   - 20pMol reverse primer (sequence ID No9)
   - 20pMol probe (sequence ID No10)
   - 3U Taq polymerase
   - nanoparticles water solution up to a final volume of 7-10µl.
□ - 66.75 µMol each precursor
   - 3,33 mMol MgCl₂
   - 20pMol forward primer (sequence ID No8)
   - 20pMol reverse primer (sequence ID No9)
   - 20pMol probe (sequence ID No10)
   - 1.5U Taq polymerase
   - nanoparticles water solution up to a final volume of 7-10µl.
□ - 66.75 µMol each precursor
   - 3,33 mMol MgCl₂
   - 20pMol forward primer (sequence ID No8)
   - 20pMol reverse primer (sequence ID No9)
   - 20pMol probe (sequence ID No10)
   - 1U Taq polymerase
   - nanoparticles water solution up to a final volume of 7-10µl.
□ - 66.75 µMol each precursor
   - 3,33 mMol MgCl₂
   - 15pMol forward primer (sequence ID No8)
   - 15pMol reverse primer (sequence ID No9)
   - 15pMol probe (sequence ID No10)
   - 3U Taq polymerase
   - nanoparticles water solution up to a final volume of 7-10µl.
□ - 66.75 µMol each precursor
   - 3,33 mMol MgCl₂
   - 15pMol forward primer (sequence ID No8)
   - 15pMol reverse primer (sequence ID No9)
   - 15pMol probe (sequence ID No10)
   - 1.5U Taq polymerase
   - nanoparticles water solution up to a final volume of 7-10µl.
□ - 66.75 µMol each precursor
   - 3,33 mMol MgCl₂
   - 15pMol forward primer (sequence ID No8)
   - 15pMol reverse primer (sequence ID No9)
   - 15pMol probe (sequence ID No10)
   - 1U Taq polymerase
   - nanoparticles water solution up to a final volume of 7-10µl.
□ - 66.75 µMol each precursor
   - 3,33 mMol MgCl₂
   - 10pMol forward primer (sequence ID No8)
   - 10pMol reverse primer (sequence ID No9)
   - 10pMol probe (sequence ID No10)
   - 1.5 U Taq polymerase
   - nanoparticles water solution up to a final volume of 7-10µl.
□ - 66.75 µMol each precursor
   - 3,33 mMol MgCl₂
   - 10pMol forward primer (sequence ID No8)
   - 10pMol reverse primer (sequence ID No9)
   - 10pMol probe (sequence ID No10)
   - 1 U Taq polymerase
   - nanoparticles water solution up to a final volume of 7-10µl.

As further alternatives for the PCR detection, the following combinations of temperatures and times for the amplification can be used.
a) first annealing step
   94 °C, 2min
   cycling sequence:
   - 94 °C: 30sec.
   - 64 °C: 45sec.
   - 72 °C: 60sec.

   total number of cycles: 40
   final elongation step
   - 72 °C: 10min.
   - 25 °C: 1min;
b) first annealing step
   94 °C, 1min
   cycling sequence:
   - 94 °C: 15sec.
   - 64 °C: 23sec.
   - 72 °C: 30sec.

   total number of cycles: 20
   final elongation step
   - 72 °C: 5min.
   - 25 °C: 30sec.
c) first annealing step
   - 94 °C,: 40sec.

   cycling sequence:
   - 94 °C: 10sec.
   - 64 °C: 15sec.
   - 72 °C: 20sec.

   total number of cycles: 13
   final elongation step
   - 72 °C: 200sec.
   - 25 °C: 20sec;
d) first annealing step
   - 94 °C,: 30sec.

   cycling sequence:
   - 94 °C: 8sec.
   - 64 °C: 11sec.
   - 72 °C: 15sec.

   total number of cycles: 10
   final elongation step
   - 72 °C: 2,5min.
   - 25 °C: 5sec.
e) first annealing step
   94 °C, 2min
   cycling sequence:
   - 94 °C: 80sec.
   - 53 °C: 20sec.
   - 72 °C: 5sec.

   total number of cycles: 35
   final elongation step
   - 72 °C: 7min.
   - 4 °C: until their removal and storage
f) first annealing step
   94 °C, 1min
   cycling sequence:
   - 94 °C: 40sec.
   - 53 °C: 10sec.
   - 72 °C: 3sec.

   total number of cycles: 18
   final elongation step
   - 72 °C: 3,5min.
   - 4 °C: until their removal and storage
g) first annealing step
   - 94 °C,: 20sec

   cycling sequence:
   - 94 °C: 27sec.
   - 53 °C: 5sec.
   - 72 °C: 2sec.

   total number of cycles: 12
   final elongation step
   - 72 °C: 2min.
   - 4 °C: until their removal and storage
h) first annealing step
   94 °C, 30sec
   cycling sequence:
   - 94 °C: 20sec.
   - 53 °C: 5sec.
   - 72 °C: 2sec.

   total number of cycles: 9
   final elongation step
   - 72 °C: 2min.
   - 4 °C: until their removal and storage
i) first annealing step
   - 94 °C,: 27sec.

   cycling sequence:
   - 94 °C: 7sec.
   - 64 °C: 10sec.
   - 72 °C: 14sec.;

   total number of cycles: 9
   final elongation step
   - 72 °C: 135sec.
   - 25 °C: 5sec;
j) first annealing step
   - 94 °C,: 24sec.

   cycling sequence:
   - 94 °C: 6sec.
   - 64 °C: 9sec.
   - 72 °C: 12sec.;

   total number of cycles: 8
   final elongation step
   - 72 °C: 2min.
   - 25 °C: 4sec.
k) first annealing step
   - 94 °C,: 30sec.

   cycling sequence:
   - 94 °C: 30sec.
   - 64 °C: 30sec.
   - 72 °C: 90sec.

   total number of cycles: 20
   final elongation step
   - 72 °C: 2,5min.
   - 25 °C: 30sec.
l) first annealing step
   - 94 °C: 30sec.

   cycling sequence:
   - 94 °C: 30sec.
   - 64 °C: 30sec.
   - 72 °C: 90sec.

   total number of cycles: 10
   final elongation step
   - 72 °C: 2,5min.
   - 25 °C: 30sec.
m) first annealing step
   - 94 °C: 30sec.

   cycling sequence:
   - 94 °C: 8sec.
   - 64 °C: 11sec.
   - 72 °C: 15sec.

   total number of cycles: 10
   final elongation step
   - 72 °C: 2,5min.
   - 25 °C: 5sec.
n) first annealing step
   - 94 °C: 30sec.

   cycling sequence:
   - 94 °C: 8sec.
   - 64 °C: 11sec.
   - 72 °C: 30sec.

   total number of cycles: 10
   final elongation step
   - 72 °C: 2,5min.
   - 25 °C: 5sec.
o) first annealing step
   - 94 °C,: 30sec.

   cycling sequence:
   - 94 °C: 8sec.
   - 64 °C: 11sec.
   - 72 °C: 45sec.

   total number of cycles: 10
   final elongation step
   - 72 °C: 2,5min.
   - 25 °C: 5sec.
p) first annealing step
   - 94 °C: 30sec.

   cycling sequence:
   - 94 °C: 8sec.
   - 64 °C: 11sec.
   - 72 °C: 60sec.

   total number of cycles: 10
   final elongation step
   - 72 °C: 2,5min.
   - 25 °C: 5sec.
q) first annealing step
   - 94 °C: 30sec.

   cycling sequence:
   - 94 °C: 8sec.
   - 64 °C: 11sec.
   - 72 °C: 12sec.

   total number of cycles: 10
   final elongation step
   - 72 °C: 2,5min.
   - 25 °C: 5sec.

Furthermore, the following combinations in temperatures and times for the ELISA and/or liquid hybridization can be used :
a) - 15µl PCR product
   - 2pmol of internal probe (Sequence ID 3- Sequence ID 7)
   - 100µl nanoparticles water solution;
   Incubation:
   - 2min. in 94 °C
   - 10min. at room temperature.
   Incubation after transfert of the mixture into microplate wells:
   22min. at 37 °C.
   Conjugated antibody incubation:
   22min. at 37 °C.
   Colorimetric substrate incubation:
   25min.;
b) - 10µl PCR product
   - 2pmol of internal probe (Sequence ID 3- Sequence ID 7)
   - 100µl nanoparticles water solution
   Incubation:
   - 2min. in 94 °C
   - 10min. at room temperature.
   Incubation after transfert of the mixture into microplate wells:
   22min. at 37 °C.
   Conjugated antibody incubation:
   22min. at 37 °C.
   Colorimetric substrate incubation:
   25min.;
c) - 15µl PCR product
   - 1pmol of internal probe (Sequence ID 3- Sequence ID 7)
   - 100µl nanoparticles water solution
   Incubation:
   - 2min. in 94 °C
   - 10min. at room temperature.
   Incubation after transfert of the mixture into microplate wells:
   22min. at 37 °C.
   Conjugated antibody incubation:
   22min. at 37 °C.
   Colorimetric substrate incubation:
   25min.;
d) - 15µl PCR product
   - 2pmol of internal probe (Sequence ID 3- Sequence ID 7)
   - 100µl nanoparticles water solution
   Incubation:
   - 2min. in 94 °C
   - 5min. at room temperature.
   Incubation after transfert of the mixture into microplate wells:
   15min. at 37 °C.
   Conjugated antibody incubation:
   15min. at 37 °C.
   Colorimetric substrate incubation:
   20min.;
e) - 15µl PCR product
   - 2pmol of internal probe (Sequence ID 3- Sequence ID 7)
   - 100µl nanoparticles water solution
   Incubation:
   - 2min. in 94 °C
   - 5min. at room temperature.
   Incubation after transfert of the mixture into microplate wells:
   10min. at 37 °C.
   Conjugated antibody incubation:
   10min. at 37 °C.
   Colorimetric substrate incubation:
   25min.;
f) - 15µl PCR product
   - 2pmol of internal probe (Sequence ID 3- Sequence ID 7)
   - 100µl nanoparticles water solution
   Incubation:
   - 2min. in 94 °C
   - 5min. at room temperature.
   Incubation after transfert of the mixture into microplate wells:
   5min. at 37 °C.
   Conjugated antibody incubation:
   10min. at 37 °C.
   Colorimetric substrate incubation:
   20min.

Another object of the invention is a kit for proceeding to the method of the invention, which comprises for the PCR detection the forward and reverse primers (amplimer forward: 5'- TTA TGA TCC TGA CTG CGA TGA -3' Sequence ID No 1; amplimer reverse: 5'- TTT TCT CTT GCT TTG TGT TTT AGT -3' Sequence ID No 2, where the forward amplimer can be labeled with biotin on one of its end or other), marked internal oligonucleotide (primers) which sequence is specified according to the gene sequence determined by the forward and reverse primers used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7: -5'-GAT CAA TAG TGA TAA CAT TAT TCT C-3') for the use in ELISA technique or/and with fluorescent probes to measure the absorbance in fluorometer, as well as dNTP, MgCl₂, Taq polymerase and nanoparticles water solution produced according to conventional protocols, see e.g. Bourlinos et al., 2003, Microporous and Mesoporous Materials, 58, 155-162; Fournaris et al., 2001, Applied Clay Science 19, 77-88; Bourlinos et al., 2003, 38, 959-963; Bournlinos and Petridis, 2001, 40, 147-151; Voulgaris and Petridis, 2002, 43, 2213-2218; Georgakilas et al., 2004, 42, 865-870), in the form of predetermined concentration and/or quantity. A further object of the invention is a kit for proceeding to the method of the invention, which comprises for the Real Time PCR detection the forward, reverse primers and probe primer (amplimer forward: 5'- TAA GGC CAA GCA GTG CAA CGG CAC C -3' Sequence ID No 8; amplimer reverse: 5'- TTT TCT CTT GCT TTG TGT TTT AGT -3' Sequence ID No 9; probe primer: 5'- FAM TCC AGT AGG TTC TCA CTC GCT CAG A' TAMRA -3' Sequence ID No 10, where the probe can be labeled with fluoroscein on one of its end or other).

Finally, still another object of the invention is the use of the method of the invention to determine whether patients suffering from malignancies of epithelium origin expressing Ep-Cam antigen after surgical removal of the tumor need or not adjuvant treatment or for monitoring the response to medical treatment in patients suffering from malignancies of epithelium origin expressing Ep-Cam antigen or for the follow up, prognosis and re-initiation of treatment of patients treated for malignancies of epithelial origin expressing Ep-Cam antigen.

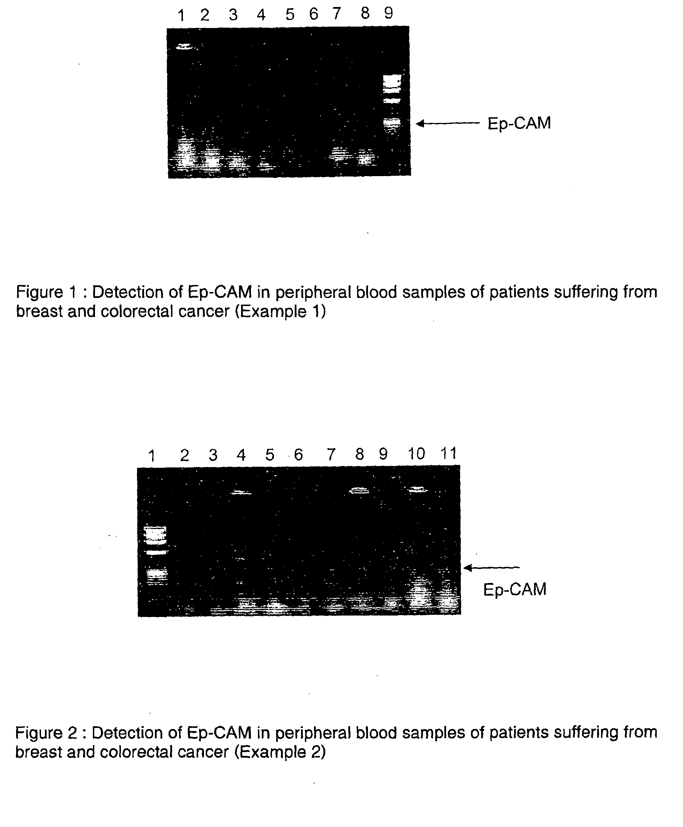

The application according to the invention will be now described in more detail with reference to the five following examples, whose results are illustrated by Figures 1 to 7.

### EXAMPLE 1

Detection of circulating micrometstatic cancer cells in peripheral blood of patients suffering from breast and colorectal cancer.

In this application according to the new invention the detection of circulating micrometastatic cancer cells was made of the determination of the expression of the Ep-CAM gene.

Total blood samples (1-2ml) treated with EDTA were taken from 15 patients suffering from breast caner and colorectal cancer at different stages of the disease independent on the treatment status; also 10 total blood samples (1-2ml) treated with EDTA were taken from healthy persons.

The total RNA was extracted from the total blood samples using well known common techniques.

A total RNA extraction can be carried out as follows: RNA extraction from 500 µl peripheral blood was performed using the TRIR reagents according to the manufacture's protocol (TRIR, Abgene, U.K.). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280. The total RNA thus extracted is then subject to a cDNA production of the complementary chain in a total volume of 20µl using specific the reverse amplimer.

The reverse primer was:

5′- TTT TCT CTT GCT TTG TGT TTT AGT –3′ (Sequence ID2)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with Nanoparticles (Bourlinos et al., 2003, Microporous and Mesoporous Materials, 58, 155-162; Fournaris et al., 2001, Applied Clay Science 19, 77-88; Bourlinos et al., 2003, 38, 959-963; Bournlinos and Petridis, 2001, 40, 147-151; Voulgaris and Petridis, 2002, 43, 2213-2218; Georgakilas et al., 2004, 42, 865-870). The reaction was incubated for 5 min in 70°C and 37°C for 30min. The reverse trancriptase was denatured at 99°C for 2.5 min and the solution cooled on ice.

The produced cDNA is then subject to a an amplification by PCR of the Ep-CAM exons marked by the forward and reverse primer in a total volume of 30µl using nanoparticles water solution.

The amplimers (primers) were:

amplimer forward: 5′- TTA TGA TCC TGA CTG CGA TGA -3′ (Sequence
ID1)

amplimer reverse: 5′- TTT TCT CTT GCT TTG TGT TTT AGT –3′
(Sequence ID2)

Amplification was performed with 5 µl of cDNA in volume of 30µl containing 3,33 mMol MgCl₂, 66,75 µMol of each precursors (d-ATP, d-CTP, d-GTP, and d-TTP, Sigma Aldrich, Germany), 30pMol forward primer, 30pMol reverse primer, 3U Taq polymerase (BioLabs, U.S.A.) and up to final volume with nanoparticles water solution. For the detection of Ep-CAM mRNA and the circulating micrometastatic cancer cells following the initial denaturation step (94°C for 1min.), 20 cycles carried out (94°C for 15sec, 64°C for 23sec, 72°C for 30sec) followed by a final elongation step at 72°C for 5min and 25 °C for 30sec.

Concerning the detection of the PCR amplicons, it can be carried out by any known appropriate method. In the present example, it was carried out by electrophoresis through an 1.5% agarose gel (Abgene, U.K.) in Tris-borate-EDTA Buffer pH=8.3 and visualised by ethidium bromide staining (Sigma Aldrich, Germany).

By using the application described above was possible to detect the Ep-CAM and the circulating micrometastatic cancer cells (Figure 1).

In the case of liquid hybridization method as final dtection system, the final PCR result analysis to detect the Ep-CAM and the circulating micrometastatic cancer cells it was carried out by ELISA with the use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'-GAT CAA TAG TGA TAA CAT TAT TCT C -3') that is conducted by adding for each reaction tube and sample:
- 15µl PCR product
- 2pmol of internal prpbe (Sequence ID 7)
- 100µl nanoparticles water solution.

Incubating the above mixture in the following combinations of temperatures and times:
2min. in 94 °C
10min. at room temperature.

The mixture will be transferred into microplate wells and incubated:
22min. at 37 °C.

The microplate wells will be washed for 3 times in time intervals of 30sec. with 300µl of PBS-T.

The conjugated anti body will be diluted in Nanoparticels in a rate depending and defined on the using conjugated antibody and will be added to the microplate wells and incubating the above mixture in the following combinations of temperatures and times:
22min. at 37 °C.

The microplate wells will be washed for 3 times in time intervals of 30sec. with 300µl of PBS-T.

In the microplate wells will be added 100µl of colorimetric substrate in a rate depending and defined on the using conjugated antibody and will be incubated for:
25min.

Following the addition of 50µl stop solution depending and defined on the using conjugated antibody and colorimetric substrate, the signal will be detected in colorimetric plate reader at extension that is dependend and defined on the using conjugated antibody and colorimetric substrate (e.g. 450nm, 405nm, 490nm, 630nm etc.).

Finally all the control samples from healthy individuals showed to be negative with both methods of final detection (gel electrophoresis and ELISA), i.e. exhibited no circulating micrometastatic cancer cells.

### EXAMPLE 2

Detection of circulating micrometstatic cancer cells in peripheral blood of patients suffering from breast and colorectal cancer.

In this application according to the new invention the detection of circulating micrometastatic cancer cells was made of the determination of the expression of the Ep-CAM gene.

Total blood samples (1-2ml) treated with EDTA were taken from 15 patients suffering from breast caner and colorectal cancer at different stages of the disease independent on the treatment status; also 10 total blood samples (1-2ml) treated with EDTA were taken from healthy persons.

The total RNA was extracted from the total blood samples using well known common techniques.

A total RNA extraction can be carried out as follows: RNA extraction from 500 µl peripheral blood was performed using the TRIR reagents according to the manufacture's protocol (TRIR, Abgene, U.K.). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280. The total RNA thus extracted is then subject to a cDNA production of the complementary chain in a total volume of 20µl using specific the reverse amplimer.

The reverse primer was:

5′- TTT TCT CTT GCT TTG TGT TTT AGT –3′ (Sequence ID2)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with nanoparticles water solution. The reaction was incubated for 5 min in 70°C and 37°C for 30min. The reverse trancriptase was denatured at 99°C for 2.5 min and the solution cooled on ice.

The produced cDNA is then subject to a an amplification by PCR of the Ep-CAM exons marked by the forward and reverse primer in a total volume of 30µl using nanoparticles water solution.

The amplimers (primers) were:

amplimer forward: 5′- TTA TGA TCC TGA CTG CGA TGA -3′ (Sequence
ID1)

amplimer reverse: 5′- TTT TCT CTT GCT TTG TGT TTT AGT –3′
(Sequence ID2)

Amplification was performed with 5 µl of cDNA in volume of 30µl containing 3,33 mMol MgCl₂, 66,75 µMol of each precursors (d-ATP, d-CTP, d-GTP, and d-TTP, Sigma Aldrich, Germany), 30pMo1 forward primer, 30pMol reverse primer, 3U Taq polymerase (BioLabs, U.S.A.) and up to final volume with nanoparticles water solution. For the detection of Ep-CAM mRNA and the circulating micrometastatic cancer cells following the initial denaturation step (94°C for 30sec.), 10 cycles carried out (94°C for 8sec, 64°C for 11sec, 72°C for 15sec) followed by a final elongation step at 72°C for 2.5min and 25 °C for 5sec.

Concerning the detection of the PCR amplicons, it can be carried out by any known appropriate method. In the present example, it was carried out by electrophoresis through an 1.5% agarose gel (Abgene, U.K.) in Tris-borate-EDTA Buffer pH=8.3 and visualised by ethidium bromide staining (Sigma Aldrich, Germany).

By using the application described above was possible to detect the Ep-CAM and the circulating micrometastatic cancer cells (Figure 2).

In the case of liquid hybridization method as final dtection system, the final PCR result analysis to detect the Ep-CAM and the circulating micrometastatic cancer cells it was carried out by ELISA with the use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'-GAT CAA TAG TGA TAA CAT TAT TCT C -3') that is conducted by adding for each reaction tube and sample:
- 10µl PCR product
- 1pmol of internal prpbe (Sequence ID 7)
- 100µl nanoparticles water solution.

Incubating the above mixture in the following combinations of temperatures and times:
2min. in 94 °C
5min. at room temperature.

The mixture will be transferred into microplate wells and incubated:
15min. at 37 °C.

The microplate wells will be washed for 3 times in time intervals of 30sec. with 300µl of PBS-T.

The conjugated anti body will be diluted in Nanoparticels in a rate depending and defined on the using conjugated antibody and will be added to the microplate wells and incubating the above mixture in the following combinations of temperatures and times:
25min. at 37 °C.

The microplate wells will be washed for 3 times in time intervals of 30sec. with 300µl of PBS-T.

In the microplate wells will be added 100µl of colorimetric substrate in a rate depending and defined on the using conjugated antibody and will be incubated for:
30min.

Following the addition of 50µl stop solution depending and defined on the using conjugated antibody and colorimetric substrate, the signal will be detected in colorimetric plate reader at extension that is dependend and defined on the using conjugated antibody and colorimetric substrate (e.g. 450nm, 405nm, 490nm, 630nm etc.).

Finally all the control samples from healthy individuals showed to be negative with both methods of final detection (gel electrophoresis and ELISA), i.e. exhibited no circulating micrometastatic cancer cells.

### EXAMPLE 3

Detection of circulating micrometstatic cancer cells in peripheral blood of patients suffering from breast and colorectal cancer.

In this application according to the new invention the detection of circulating micrometastatic cancer cells was made of the determination of the expression of the Ep-CAM gene.

Total blood samples (1-2ml) treated with EDTA were taken from 15 patients suffering from breast caner and colorectal cancer at different stages of the disease independent on the treatment status; also 10 total blood samples (1-2ml) treated with EDTA were taken from healthy persons.

The total RNA was extracted from the total blood samples using well known common techniques.

A total RNA extraction can be carried out as follows: RNA extraction from 500 µl peripheral blood was performed using the TRIR reagents according to the manufacture's protocol (TRIR, Abgene, U.K.). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280.

The total RNA thus extracted is then subject to a cDNA production of the complementary chain in a total volume of 20µl using specific the reverse amplimer.

The reverse primer was:

5′- TTT TCT CTT GCT TTG TGT TTT AGT –3′ (Sequence ID2)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with nanoparticles water solution. The reaction was incubated for 5 min in 70°C and 37°C for 30min. The reverse trancriptase was denatured at 99°C for 2.5 min and the solution cooled on ice.

The produced cDNA is then subject to a an amplification by PCR of the Ep-CAM exons marked by the forward and reverse primer in a total volume of 15µl using nanoparticles water solution.

The amplimers (primers) were:

amplimer forward: 5′- TTA TGA TCC TGA CTG CGA TGA -3′ (Sequence
ID1)

amplimer reverse: 5´- TTT TCT CTT GCT TTG TGT TTT AGT –3´
(Sequence ID2)

Amplification was performed with 5 µl of cDNA in volume of 15µl containing 66,75 µMol of each precursors (d-ATP, d-CTP, d-GTP, and d-TTP, Sigma Aldrich, Germany), 10pMol forward primer, 10pMol reverse primer, 3U Taq polymerase (BioLabs, U.S.A.) and up to final volume with nanoparticles water solution. For the detection of Ep-CAM mRNA and the circulating micrometastatic cancer cells following the initial denaturation step (94°C for 30sec.), 10 cycles carried out (94°C for 30sec, 64°C for 30sec, 72°C for 90sec) followed by a final elongation step at 72°C for 10min and 25 °C for 1min.

Concerning the detection of the PCR amplicons, it can be carried out by any known appropriate method. In the present example, it was carried out by electrophoresis through an 1.5% agarose gel (Abgene, U.K.) in Tris-borate-EDTA Buffer pH=8.3 and visualised by ethidium bromide staining (Sigma Aldrich, Germany).

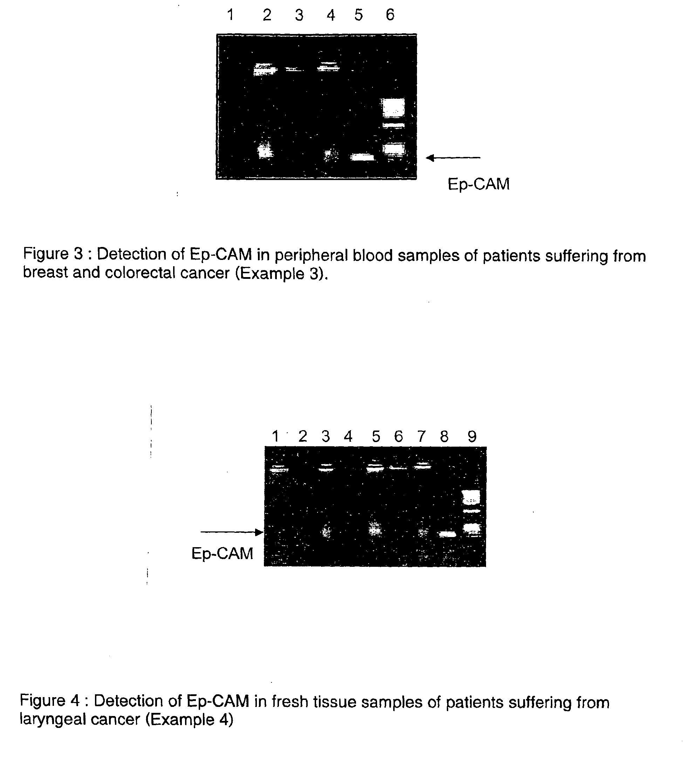

By using the application described above was possible to detect the Ep-CAM and the circulating micrometastatic cancer cells (Figure 3).

In the case of liquid hybridization method as final dtection system, the final PCR result analysis to detect the Ep-CAM and the circulating micrometastatic cancer cells it was carried out by ELISA with the use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'-GAT CAA TAG TGA TAA CAT TAT TCT C -3') that is conducted by adding for each reaction tube and sample:
- 15µl PCR product
- 2pmol of internal prpbe (Sequence ID 5)
- 100µl nanoparticles water solution.

Incubating the above mixture in the following combinations of temperatures and times:
2min. in 94 °C
10min. at room temperature.

The mixture will be transferred into microplate wells and incubated:
10min. at 37 °C.

The microplate wells will be washed for 3 times in time intervals of 30sec. with 300µl of PBS-T.

The conjugated anti body will be diluted in Nanoparticels in a rate depending and defined on the using conjugated antibody and will be added to the microplate wells and incubating the above mixture in the following combinations of temperatures and times:
22min. at 37 °C.

The microplate wells will be washed for 3 times in time intervals of 30sec. with 300µl of PBS-T.

In the microplate wells will be added 100µl of colorimetric substrate in a rate depending and defined on the using conjugated antibody and will be incubated for:
25min.

Following the addition of 50µl stop solution depending and defined on the using conjugated antibody and colorimetric substrate, the signal will be detected in colorimetric plate reader at extension that is dependend and defined on the using conjugated antibody and colorimetric substrate (e.g. 450nm, 405nm, 490nm, 630nm etc.).

Finally all the control samples from healthy individuals showed to be negative with both methods of final detection (gel electrophoresis and ELISA), i.e. exhibited no circulating micrometastatic cancer cells.

### EXAMPLE 4

### Detection of micrometstatic cancer cells in tissues of patients suffering from laryngeal cancer.

In this application according to the new invention the detection of micrometastatic cancer cells was made of the determination of the expression of the Ep-CAM gene.

Tissue samples were taken from 20 patients suffering from laryngeal cancer at different stages of the disease independent on the treatment status; also 10 total blood samples (1-2ml) treated with EDTA were taken from healthy persons.

The total RNA was extracted from the tissue samples using well known common techniques.

A total RNA extraction can be carried out as follows: RNA extraction from tissues was performed using the TRIR reagents according to the manufacture's protocol (TRIR, Abgene, U.K.) following homogenization and for total blood samples according to the manufacture's protocol (TRI, Sigma Aldrich, Germany). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280.

The total RNA thus extracted is then subject to a cDNA production of the complementary chain in a total volume of 20µl using specific the reverse amplimer.

The reverse primer was:

5′- TTT TCT CTT GCT TTG TGT TTT AGT –3′ (Sequence ID2)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with nanoparticles water solution. The reaction was incubated for 5 min in 70°C and 37°C for 20min. The reverse trancriptase was denatured at 99°C for 2.5 min and the solution cooled on ice.

The produced cDNA is then subject to amplification by PCR of the Ep-CAM exons marked by the forward and reverse primer in a total volume of 15µl using nanoparticles water solution.

The amplimers (primers) were:

amplimer forward: 5´- TTA TGA TCC TGA CTG CGA TGA -3´ (Sequence
ID1)

amplimer reverse: 5´- TTT TCT CTT GCT TTG TGT TTT AGT –3´
(Sequence ID2)

Amplification was performed with 5 µl of cDNA in volume of 15µl containing 66.75 µMol of each precursors (d-ATP, d-CTP, d-GTP, and d-TTP, Sigma Aldrich, Germany), 1pMol forward primer, 1pMol reverse primer, 3U Taq polymerase (BioLabs, U.S.A.) and up to final volume with nanoparticles water solution. For the detection of Ep-CAM mRNA and the circulating micrometastatic cancer cells following the initial denaturation step (94°C for 30sec.), 10 cycles carried out (94°C for 30sec, 64°C for 30sec, 72°C for 90sec) followed by a final elongation step at 72°C for 10min and 25 °C for 1min.

Concerning the detection of the PCR amplicons, it can be carried out by any known appropriate method. In the present example, it was carried out by electrophoresis through an 1.5% agarose gel (Abgene, U.K.) in Tris-borate-EDTA Buffer pH=8.3 and visualised by ethidium bromide staining (Sigma Aldrich, Germany).

By using the application described above was possible to detect the Ep-CAM and the circulating micrometastatic cancer cells (Figure 4).

In the case of liquid hybridization method as final dtection system, the final PCR result analysis to detect the Ep-CAM and the circulating micrometastatic cancer cells it was carried out by ELISA with the use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'-GAT CAA TAG TGA TAA CAT TAT TCT C -3') that is conducted by adding for each reaction tube and sample:
- 1-15µl PCR product
- 0,2-2pmol of internal prpbe (Sequence ID 6)
- 100µl nanoparticles water solution.

Incubating the above mixture in the following combinations of temperatures and times:
2min. in 94 °C
10min. at room temperature.

The mixture will be transferred into microplate wells and incubated:
22min. at 37 °C.

The microplate wells will be washed for 3 times in time intervals of 30sec. with 300µl of PBS-T.

The conjugated anti body will be diluted in Nanoparticels in a rate depending and defined on the using conjugated antibody and will be added to the microplate wells and incubating the above mixture in the following combinations of temperatures and times:
22min. at 37 °C.

The microplate wells will be washed for 3 times in time intervals of 30sec. with 300µl of PBS-T.

In the microplate wells will be added 100µl of colorimetric substrate in a rate depending and defined on the using conjugated antibody and will be incubated for:
25min.

Following the addition of 50µl stop solution depending and defined on the using conjugated antibody and colorimetric substrate, the signal will be detected in colorimetric plate reader at extension that is dependend and defined on the using conjugated antibody and colorimetric substrate (e.g. 450nm, 405nm, 490nm, 630nm etc.).

Finally all the control samples from healthy individuals showed to be negative with both methods of final detection (gel electrophoresis and ELISA), i.e. exhibited no circulating micrometastatic cancer cells.

### EXAMPLE 5

### Detection of micrometstatic cancer cells in tissues of patients suffering from laryngeal cancer.

In this application according to the new invention the detection of micrometastatic cancer cells was made of the determination of the expression of the Ep-CAM gene.

Tissue samples were taken from 20 patients suffering from laryngeal cancer at different stages of the disease independent on the treatment status; also 10 total blood samples (1-2ml) treated with EDTA were taken from healthy persons.

The total RNA was extracted from the tissue samples using well known common techniques.

A total RNA extraction can be carried out as follows: RNA extraction from tissues was performed using the TRIR reagents according to the manufacture's protocol (TRIR, Abgene, U.K.) following homogenization and for total blood samples according to the manufacture's protocol (TRI, Sigma Aldrich, Germany). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA.

After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water.

The quality and quantity of RNA was assessed by a spectrophotometer reading at 260/280.

The total RNA thus extracted is then subject to a cDNA production of the complementary chain in a total volume of 20µl using specific the reverse amplimer.

The reverse primer was:

5´- TTT TCT CTT GCT TTG TGT TTT AGT –3´ (Sequence ID2)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with nanoparticles water solution. The reaction was incubated for 5 min in 70°C and 37°C for 20min. The reverse trancriptase was denatured at 99°C for 2.5 min and the solution cooled on ice.

The produced cDNA is then subject to amplification by PCR of the Ep-CAM exons marked by the forward and reverse primer in a total volume of 30µl using nanoparticles water solution.

The amplimers (primers) were:

amplimer forward: 5´- TTA TGA TCC TGA CTG CGA TGA -3´ (Sequence
ID1)

amplimer reverse: 5′- TTT TCT CTT GCT TTG TGT TTT AGT –3′
(Sequence ID2)

Amplification was performed with 5 µl of cDNA in volume of 30µl containing 66.75 µMol of each precursors (d-ATP, d-CTP, d-GTP, and d-TTP, Sigma Aldrich, Germany), 100fMol forward primer, 100fMol reverse primer, 3U Taq polymerase (BioLabs, U.S.A.) and and up to final volume with nanoparticles water solution. For the detection of Ep-CAM mRNA and the circulating micrometastatic cancer cells following the initial denaturation step (94°C for 30sec.), 10 cycles carried out (94°C for 30sec, 64°C for 30sec, 72°C for 90sec) followed by a final elongation step at 72°C for 10min and 25 °C for 1min.

Concerning the detection of the PCR amplicons, it can be carried out by any known appropriate method. In the present example, it was carried out by electrophoresis through an 1.5% agarose gel (Abgene, U.K.) in Tris-borate-EDTA Buffer pH=8.3 and visualised by ethidium bromide staining (Sigma Aldrich, Germany).

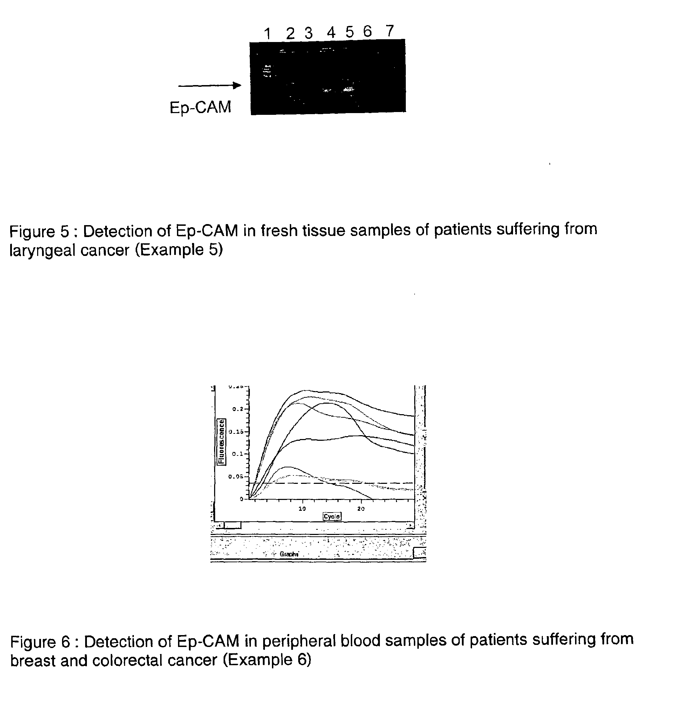

By using the application described above was possible to detect the Ep-CAM and the micrometastatic cancer cells (Figure 5).

In the case of liquid hybridization method as final dtection system, the final PCR result analysis to detect the Ep-CAM and the circulating micrometastatic cancer cells it was carried out by ELISA with the use of an internal probe primer which sequence is specified according to the gene sequence determined by the forward and reverse primer used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7:- 5'-GAT CAA TAG TGA TAA CAT TAT TCT C -3') that is conducted by adding for each reaction tube and sample:
- 15µl PCR product
- 2pmol of internal prpbe (Sequence ID 4)
- 100µl nanoparticles water solution.

Incubating the above mixture in the following combinations of temperatures and times:
- 2min. in 94 °C
- 10min. at room temperature.

The mixture will be transferred into microplate wells and incubated:
- 20min. at 37 °C.

The microplate wells will be washed for 3 times in time intervals of 30sec. with 300µl of PBS-T.

The conjugated anti body will be diluted in Nanoparticels in a rate depending and defined on the using conjugated antibody and will be added to the microplate wells and incubating the above mixture in the following combinations of temperatures and times:
- 20min. at 37 °C.

The microplate wells will be washed for 3 times in time intervals of 30sec. with 300µl of PBS-T.

In the microplate wells will be added 100µl of colorimetric substrate in a rate depending and defined on the using conjugated antibody and will be incubated for:
- 25min.

Following the addition of 50µl stop solution depending and defined on the using conjugated antibody and colorimetric substrate, the signal will be detected in colorimetric plate reader at extension that is dependend and defined on the using conjugated antibody and colorimetric substrate (e.g. 450nm, 405nm, 490nm, 630nm etc.).

Finally all the control samples from healthy individuals showed to be negative with both methods of final detection (gel electrophoresis and ELISA), i.e. exhibited no circulating micrometastatic cancer cells.

### EXAMPLE 6

### Detection of circulating micrometstatic cancer cells in peripheral blood of patients suffering from breast and colorectal cancer.

In this application according to the new invention the detection of circulating micrometastatic cancer cells was made of the determination of the expression of the Ep-CAM gene.

Total blood samples (1-2ml) treated with EDTA were taken from 7 patients suffering from breast caner and colorectal cancer at different stages of the disease independent on the treatment status; also 10 total blood samples (1-2ml) treated with EDTA were taken from healthy persons.

The total RNA was extracted from the total blood samples using well known common techniques.

A total RNA extraction can be carried out as follows: RNA extraction from 500 µl peripheral blood was performed using the TRIR reagents according to the manufacture's protocol (TRIR, Abgene, U.K.). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280. The total RNA thus extracted is then subject to a cDNA production of the complementary chain in a total volume of 20µl using specific the reverse amplimer.

The reverse primer was:

5´- TTT TCT CTT GCT TTG TGT TTT AGT –3´ (Sequence ID9)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with Nanoparticles (Bourlinos et al., 2003, Microporous and Mesoporous Materials, 58, 155-162; Fournaris et al., 2001, Applied Clay Science 19, 77-88; Bourlinos et al., 2003, 38, 959-963; Bournlinos and Petridis, 2001, 40, 147-151; Voulgaris and Petridis, 2002, 43, 2213-2218; Georgakilas et al., 2004, 42, 865-870). The reaction was incubated for 5 min in 70°C and 37°C for 30min. The reverse trancriptase was denatured at 99°C for 2.5 min and the solution cooled on ice.

The produced cDNA is then subject to an amplification by Real Time PCR of the Ep-CAM exons marked by the forward and reverse primer in a total volume of 15µl using nanoparticles water solution.

The amplimers (primers) were:

amplimer forward: 5´- TAA GGC CAA GCA GTG CAA CGG CAC C -3´
(Sequence ID8)

amplimer reverse: 5´- TTT TCT CTT GCT TTG TGT TTT AGT –3´
(Sequence ID9)

amplimer probe: 5′- FAM TCC AGT AGG TTC TCA CTC GCT CAG A’
TAMRA –3′ (Sequence ID10).

Amplification was performed with 5 µl of cDNA in volume of 15µl containing 66,75 µMol of each precursors (d-ATP, d-CTP, d-GTP, and d-TTP, Sigma Aldrich, Germany), 30pMol forward primer, 30pMol reverse primer, 30pMol probe primer 1,5U Taq polymerase (BioLabs, U.S.A.) and up to final volume with nanoparticles water solution. For the detection of Ep-CAM mRNA and the circulating micrometastatic cancer cells following the initial denaturation step (94°C for 2sec.) 13 cycles is carried out (94°C for 15sec, 62°C for 30sec).

By using the application described above was possible to detect the Ep-CAM and the circulating micrometastatic cancer cells (Figure 6).

### EXAMPLE 7

### Detection of circulating micrometstatic cancer cells in peripheral blood of patients suffering from breast and colorectal cancer.

In this application according to the new invention the detection of circulating micrometastatic cancer cells was made of the determination of the expression of the Ep-CAM gene.

Total blood samples (1-2ml) treated with EDTA were taken from 6 patients suffering from breast caner and colorectal cancer at different stages of the disease independent on the treatment status; also 10 total blood samples (1-2ml) treated with EDTA were taken from healthy persons.

The total RNA was extracted from the total blood samples using well known common techniques.

A total RNA extraction can be carried out as follows: RNA extraction from 500 µl peripheral blood was performed using the TRIR reagents according to the manufacture's protocol (TRIR, Abgene, U.K.). Subsequently, each sample was incubated for 10 minutes following the addition of 750 µl of TRIR reagent on ice. RNA was then extracted by the addition of 250 µl of chloroform. The tube was vortexed for 5 min and then placed on ice for 10 min. The phases were separated by a 15 min centrifugation at 11,000g in a table-top microcentrifuge at 4°C. The aqueous phase was transferred to a sterile tube, an equal volume of isopropanol was added and the mixture was incubated on ice for 10 min to precipitate RNA. After another 10 min centrifugation at 11,000g in a table-top microcentrifuge at 4°C, the supernatant was carried out. One ml of 75% frozen ethanol was added to the tube and recentrifuged at 4°C for 5 min. The contents of the tube were dried (5-10 min) at room temperature. The RNA should not dry completely before re-suspension in 40µl diethyl-pyrocarbonate-treated water. The quality and quantity of RNA was assessed by a spectrophotometer reading at 260/280.

The total RNA thus extracted is then subject to a cDNA production of the complementary chain in a total volume of 20µl using specific the reverse amplimer.

The reverse primer was:

5′- TTT TCT CTT GCT TTG TGT TTT AGT –3′ (Sequence ID9)

The Reverse transcription was performed using the protocol according to the new invention. First strand c-DNA synthesis was done with 100pMol reverse primer in a 20µl reaction, which contains 1µg of RNA after spectrophotometer analysis, 125µMol of each precursor (d-ATP, d-CTP, d-GTP, and d-TTP, Sigma Aldrich, Germany), 20U Rnase inhibitor (Sigma Aldrich, Germany), 125U reverse transcriptase (Sigma Aldrich, Germany) and up to the final volume with Nanoparticles (Bourlinos et al., 2003, Microporous and Mesoporous Materials, 58, 155-162; Fournaris et al., 2001, Applied Clay Science 19, 77-88; Bourlinos et al., 2003, 38, 959-963; Bournlinos and Petridis, 2001, 40, 147-151; Voulgaris and Petridis, 2002, 43, 2213-2218; Georgakilas et al., 2004, 42, 865-870). The reaction was incubated for 5 min in 70°C and 37°C for 30min. The reverse trancriptase was denatured at 99°C for 2.5 min and the solution cooled on ice.

The produced cDNA is then subject to an amplification by Real Time PCR of the Ep-CAM exons marked by the forward and reverse primer in a total volume of 15µl using nanoparticles water solution.

The amplimers (primers) were:

amplimer forward: 5′- TAA GGC CAA GCA GTG CAA CGG CAC C -3′
(Sequence ID8)

amplimer reverse: 5′- TTT TCT CTT GCT TTG TGT TTT AGT –3′
(Sequence ID9)

amplimer probe: 5′- FAM TCC AGT AGG TTC TCA CTC GCT CAG A’
TAMRA –3′ (Sequence ID10).

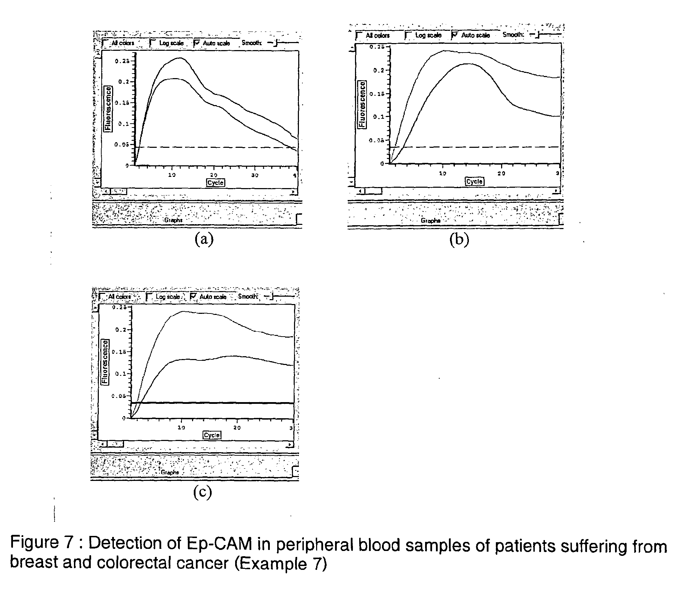

Amplification was performed with 5 µl of cDNA in volume of 15µl containing 66,75 µMol of each precursors (d-ATP, d-CTP, d-GTP, and d-TTP, Sigma Aldrich, Germany), 20pMol forward primer, 20pMol reverse primer, 20pMol probe primer 1,5U Taq polymerase (BioLabs, U.S.A.) and up to final volume with nanoparticles water solution. For the detection of Ep-CAM mRNA and the circulating micrometastatic cancer cells following the initial denaturation step (94°C for 2sec.) 13 cycles is carried out (94°C for 15sec, 62°C for 30sec). By using the application described above was possible to detect the Ep-CAM and the circulating micrometastatic cancer cells (Figure 7).

The new invention decreases dramatically the time and the quantity of the reagents, that are sufficient to carry out the detection or a test of the Ep-CAM sequences and the identification of circulating micrometastatic cancer cells in peripheral blood or/and tissues and any other body fluids of patients suffering from epithelial cancer, using nanoparticles water solution, in PCR and/or RT-PCR applications, without simultaneously the use of any buffer and buffer system for the RT- and PCR reaction, as well as magnesium chloride (MgCl₂) or eventually the reverse primer.

The present invention is constituted and characterized through the complete procedure from various elements:
**A**) In the procedure for the complementary DNA (cDNA) production is not used any buffer and specific proceeded water but nanoparticles water solution.
**B**) In the procedure for the complementary DNA (cDNA) production is used new accorduing the new invention protocol procedure and the time needed for the cDNA production is reduced up to 50% and 80% in comparison with any it is until today described in the international scientific literature.
**C**) In the procedure for the complementary DNA (cDNA) production is decreased the volume and concentration of using reagents and it is not used any DTT in comparison with any it is until today described in the international scientific literature. Furthermore, the application of specific protocol procedure using nanoparticles water solution, reagent concentration and thermical incubation profile for the cDNA production using the extracted total RNA, the produced cDNA from the total RNA, which is extracted from the peripheral blood or tissues (and any other body fluids) of patient suffering from cancer epithelial origin with any common method, is constituted only and specific in molecules expressed from the Ep-CAM gene.
**D**) Modifies and applies the new procedure and thermical protocols for the PCR reaction.
**E**) It is not used, as described above, for the PCR reaction any buffer or buffer system and magnesium chloride (MgCl₂) in comparison with any it is until today described in the international scientific literature.
**F**) It is not used, as described above, and in comparison with any it is until today described in the international scientific literature, for the PCR reaction any PCR-water but nanoparticles water solution.
**G**) It is reduced dramatically as described above, and in comparison with any it is until today described in the international scientific literature, the concentration and the quantity of reagents that are used to the PCR reaction.
**H**) It is not used, as described above, and in comparison with any it is until today described in the international scientific literature, for the PCR reaction the reverse primer.
**I**) It is reduced dramatically as described above, and in comparison with any it is until today described in the international scientific literature, the concentration and the quantity of the Taq polymerase enzyme
**J**) It is reduced dramatically as described above, in few minutes lasting the total time and the PCR cycles are needed (up to 12 min.), a reduction that reaches up to 85% that it is until today described in the international scientific literature for the application of the PCR reaction.

### Description of the annexed Figures :

- Figure 1: Detection of Ep-CAM in peripheral blood samples of patients suffering from breast and colorectal cancer according to the procedure described in Example 1 :
   Blood samples 1, 2 and 7 are positive for the expression of Ep-CAM according to the procedure described in Example 1. Blood sample 3 -6 and 8 are from healthy individuals and negative for the EpCAM expression. Lane 9 is length marker.
- Figure 2: Detection of Ep-CAM in peripheral blood samples of patients suffering from breast and colorectal cancer according to the procedure described in Example 2 :
   Blood samples 10 and 11 are positive for the expression of Ep-CAM according to the procedure described in Example 2. Blood sample 2- 9 are from healthy individuals and negative for the EpCAM expression. Lane 1 is length marker.
- Figure 3: Detection of Ep-CAM in peripheral blood samples of patients suffering from breast and colorectal cancer according to the procedure described in Example 3 :
   Blood samples 2 - 5 positive for the expression of Ep-CAM according to the procedure described in Example 3. Blood sample 1 is from healthy individual and negative for the EpCAM expression. Lane 6 is length marker.
- Figure 4: Detection of Ep-CAM in fresh tissue samples of patients suffering from laryngeal cancer according to the procedure described in Example 4 :
   Fresh laryngeal cancer tissue samples 1-3 and 5-9 are positive for the expression of Ep-CAM according to the procedure described in Example 4. Tissue sample 4 is negative for the EpCAM expression. Lane 9 is length marker.
- Figure 5: Detection of Ep-CAM in fresh tissue samples of patients suffering from laryngeal cancer according to the procedure described in example 5
   Fresh laryngeal cancer tissue samples 2-5 are positive for the expression of Ep-CAM according to the procedure described in Example 5. Tissue sample 6 is negative for the EpCAM expression. Lane 7 is positive control for the Ep-CAM expression and lane 1 is length marker.
- Figure 6: Detection of Ep-CAM in peripheral blood samples of patients suffering from breast and colorectal cancer according to the procedure described in Example 6 :
   Blood samples positive for the expression of Ep-CAM according to the procedure described in Example 6 (positive sample above to the perforated line). Blood sample from healthy individuals are negative for the EpCAM expression (brown line).
- Figure 7: Detection of Ep-CAM in peripheral blood samples of patients suffering from breast and colorectal cancer according to the procedure described in Example 7 :
   Blood samples positive for the expression of Ep-CAM according to the procedure described in Example 7 (positive sample above to the perforated line). Blood sample from healthy individuals are negative for the EpCAM expression (a: green and yellow line; b: blue line and c: blue line).

## Claims

1. Method for the detection of cancer cells of epithelium origin expressing the Ep-Cam antigen and identification of circulating micrometastatic cancer cells in peripheral blood tissues and other body fluids of patients suffering from cancer of epithelial origin, using a sample of total blood, body fluids or tissue, comprising the amplification of a cDNA molecule encoding the Ep-CAM antigen, which includes the following steps:
(i) hybridizing mRNA isolated by any conventional technique, with a specific (reverse primer) oligonucleotide that hybridizes specifically to the nucleotide sequence of the Ep-CAM antigen;
(ii) producing sequence specific single stranded DNA (complementary DNA) with or without 0.01Mol DTT with the use of reverse transcriptase from mRNA extracted by any conventional technique, as follows:
(a) for each sample preparing a reaction mixture of 10µl end volume comprising :
- 125µMol of each precursor
- 10-40U ribonuclease inhibitor
- 125-250U reverse transcriptase enzyme, and
- nanoparticles water solution up to final volume of 10 µl;
(b) adding in a reaction tube :
- 10-100pMol of reverse primer (sequence ID No2)
- 1-2µl of total RNA concentrated 1µg/µl, and
- nanoparticles water solution up to fulfillment of the above volume;
(c) placing the mixture including the reverse primer on a thermal cycler, and:
- incubating at 70 °C for 5minutes,
- placing the tubes on ice,
- heating the cycler at 37 °C and maintaining this temperature,
- adding in the tubes that are placed on ice 10µl of the reaction mixture
and incubating at :
37 °C, for 10-30min.
95 °C for 2-5 minutes for the inactivation of the reverse transcriptase enzyme;
(iii) detecting Ep-CAM and identifying circulating metastatic cancer cells.

2. Method according to claim 1, comprising detecting Ep-CAM and identifying circulating micrometastatic cancer cells either (A) by carrying out PCR reaction, performed under specific conditions with the use of 5µl of the above produced cDNA, nanoparticles water solution, specific pairs of oligonucleotides (primers) that hybridize specifically to the nucleotide sequence of the Ep-CAM antigen mRNA (amplimer forward: 5'- TTA TGA TCC TGA CTG CGA TGA-3' Sequence ID No 1; amplimer reverse: 5'- TTT TCT CTT GCT TTG TGT TTT AGT -3' Sequence ID No 2, where the forward amplimer can be labeled with biotin on one of its end or other) and allows the DNA Taq polymerase to amplify a huge amount of DNA fragments originated from the specific primer pairs used, as follows :
(d) proceeding to the PCR reaction with or without magnesium chloride (MgCl₂) and with or without the reverse primer (amplimer) by adding for each reaction tube and sample :
- 66.75 µMol of each precursor
- 50fMol-30pMol of forward primer (sequence ID No1)
- 50fMol-30pMol of reverse primer (sequence ID No2)
- 0.6-6U of Taq polymerase, and
- nanoparticles water solution up to a final volume of 10-30µl;
(e) incubating the above mixture according to a prototype thermical protocol in thermocycler, which is consisting in one of the two following combinations of temperatures and times:
(1)
- first annealing step
94 °C, 24sec-2min.
- cycling sequence:
94 °C 6-30sec.
64 °C 9-45sec.
72 °C 12-90sec.
total number of cycles: 8-40
- final elongation step
72 °C 2-10min.
25 °C 4sec.-1min.
or (2)
- first annealing step
94 °C, 30sec - 2min.
- cycling sequence:
94 °C 20sec.-80sec.
53 °C 5sec.- 30sec.
72 °C 10sec.- 90sec.
total number of cycles: 9 -35
- final elongation step
72 °C 2min.- 7min.
4 °C until their removal and storage;
(f) conducting the final PCR result analysis;
or (B) by carrying out Real Time PCR reaction, performed under specific conditions with the use of 5µl of the above produced cDNA, nanoparticles water solution, specific pairs of oligonucleotides (primers) that hybridize specifically to the nucleotide sequence of the Ep-CAM antigen mRNA using a specific probe primer (amplimer forward: 5'- TAA GGC CAA GCA GTG CAA CGG CAC C -3' Sequence ID No 8; amplimer reverse: 5'- TTT TCT CTT GCT TTG TGT TTT AGT -3' Sequence ID No 9; amplimer probe: 5'-FAM TCC AGT AGG TTC TCA CTC GCT CAG A' TAMRA -3' Sequence ID No 10, where the probe can be labeled with fluoroscein on one of its end or other) and allows the DNA Taq polymerase to amplify a huge amount of DNA fragments originated from the specific primer pairs used, as follows :
g) prepare specific cDNA for the gene for Ep-Cam using the cDNA protocol described above with the use of the Real Time PCR specific amplimer reverse (Sequence ID No9);
(h) proceeding to the Real Time PCR reaction with or without magnesium chloride (MgCl₂) and with or without the reverse primer (amplimer) by adding for each reaction tube and sample :
- 66.75 µMol of each precursor
- 10pMol-30pMol of forward primer (sequence ID No8)
- 10pMol-30pMol of reverse primer (sequence ID No9)
- 10pMol-30pMol of probe primer (sequence ID No10)
- 1-3U of Taq polymerase, and
- nanoparticles water solution up to a final volume of 7-10µl;
(i) incubating the above mixture according to a prototype thermical protocol in Real Time PCR thermocycler, which is consisting in the following combinations of temperatures and times:
- first annealing step
94 °C, 2sec
.
- cycling sequence:
94 °C 15sec.
62 °C 30sec.
total number of cycles: 13
10 °C until their removal;
(j) conducting the final Real Time PCR result analysis.

3. Method according to claim 2, in which the final PCR result analysis is carried out according to one of the following techniques :
i) agarose gel electrophoresis and staining of the PCR products with ethidium bromide, with final visualization under UV radiation and their grabbing;
ii) use of an internal probe primer which sequence is specified according to the gene sequence determined by the forward and reverse primer used in PCR (such as Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7: -5'-GAT CAA TAG TGA TAA CAT TAT TCT C-3'), under the application of any hybridization technique on membrane or in liquids;
iii) marked oligonucleotides (primers) for use in ELISA technique;
iv) marked oligonucleotides (primers) with fluorescent probes to measure the absorbance in fluorometer; or,
v) marked internal oligonucleotide (primers) whose sequence is specified according to the gene sequence determined by the forward and reverse primer used in PCR (such as Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7: -5'-GAT CAA TAG TGA TAA CAT TAT TCT C-3') for the use in real time PCR or/and in ELISA technique or/and with fluorescent probes to measure the absorbance in fluorometer, and
vi) in case of Real Time PCR detection, the use of nanoparticles water solution, specific pairs of oligonucleotides (primers) that hybridize specifically to the nucleotide sequence of the Ep-CAM antigen mRNA using a specific probe primer (amplimer forward: 5'- TAA GGC CAA GCA GTG CAA CGG CAC C -3' Sequence ID No 8; amplimer reverse: 5'- TTT TCT CTT GCT TTG TGT TTT AGT -3' Sequence ID No 9; amplimer probe: 5'- FAM TCC AGT AGG TTC TCA CTC GCT CAG A' TAMRA -3' Sequence ID No 10, where the probe can be labeled with fluoroscein on one of its end or other).

4. Method according to claim 3, in which the final PCR result analysis is carried out by using the technique of liquid hybridization, it will be then conducted by adding for each reaction tube and sample :
- 1-15µl PCR product
- 0,2-2pmol of internal probe (Sequence ID 2-Sequence ID 7)
- 100 µl nanoparticles water solution;
incubating the above mixture in the following combinations of temperatures and times :
2-5min. in 94°C,
5-10min. at room temperature;
transferring the mixture into microplate wells and incubating 5-25min. at 37°C;
washing the microplate wells for 3 times in time intervals of 30sec. with 300µl of PBS-T;
diluting the conjugated antibody in nanoparticles water solution in a rate depending and defined on the using conjugated antibody, and adding to the microplate wells and incubating the above mixture in the following combinations of temperatures and times :
5-25min. at 37°C;
washing the microplate wells for 3 times in time intervals of 30sec. With 300µl of PBS-T;
adding in the microplate wells 100µl of colorimetric substrate in a rate depending and defined on the using conjugated antibody, and incubating for 5-30min.;
and following the addition of 50µl stop solution depending and defined on the using conjugated antibody and colorimetric substrate, detecting the signal in colorimetric plate reader at extension that is dependent and defined on the using conjugated antibody and colorimetric substrate (e.g. 450nm, 405nm, 490nm, 630nm, etc.).

5. Method according to claim 3, in which the final PCR result analysis is carried out by using the technique of ELISA, it will be then conducted by adding for each reaction tube and sample :
- 1-15µl PCR product
- 0,2-2pmol of internal probe (Sequence ID 3-Sequence ID 7)
- 100µl nanoparticles water solution;
incubating the above mixture in the following combinations of temperatures and times:
2-5min. in 94 °C
5-10min. at room temperature;
transferring the mixture into microplate wells and incubating
5-25min. at 37 °C;
washing the microplate wells for 3 times in time intervals of 30sec. with 300µl of PBS-T;
diluting the conjugated antibody in nanoparticles water solution in a rate depending and defined on the using conjugated antibody, and adding to the microplate wells and incubating the above mixture in the following combinations of temperatures and times:
5-25min. at 37 °C;
washing the microplate wells for 3 times in time intervals of 30sec. with 300µl of PBS-T;
adding in the microplate wells 100µl of colorimetric substrate in a rate depending and defined on the using conjugated antibody and will be incubated for 5-30min.; and
following the addition of 50µl stop solution depending and defined on the using conjugated antibody and colorimetric substrate, detecting the signal in colorimetric plate reader at extension that is dependent and defined on the using conjugated antibody and colorimetric substrate (e.g. 450nm, 405nm, 490nm, 630nm, etc.).

6. Method according to one of claims 2 to 5, in which one of the following compositions with or without magnesium chloride (MgC12) or with or without reverse primer is used :
a) For the PCR detection:
□ - 66.75 µMol each precursor
- 3,33 mMol MgCl₂
- 30pMol forward primer (sequence ID No1)
- 30pMol reverse primer (sequence ID No2)
- 0,6U Taq polymerase
- nanoparticles water solution up to a final volume of 10-30µl.
□ - 66.75 µMol each precursor
- 30pMol forward primer (sequence ID No1)
- 30pMol reverse primer (sequence ID No2)
- 0,6U Taq polymerase
- nanoparticles water solution up to a final volume of 10-30µl.
□ - 66.75 µMol each precursor
- 3,33 mMol MgCl₂
- 10pMol forward primer (sequence ID No1)
- 10pMol reverse primer (sequence ID No2)
- 0,6U Taq polymerase
- nanoparticles water solution up to a final volume of 1-30µl.
□ - 66.75 µMol each precursor
- 10pMol forward primer (sequence ID No1)
- 10pMol reverse primer (sequence ID No2)
- 0,6U Taq polymerase
- nanoparticles water solution up to a final volume of 10-30µl.
□ - 66.75 µMol each precursor
- 3,33 mM MgCl₂
- 5pMol forward primer (sequence ID No1)
- 5pMol reverse primer (sequence ID No2)
- 0,6U Taq polymerase
- nanoparticles water solution up to a final volume of 10-30µl.
□ - 66.75 µMol each precursor
- 5pMol forward primer (sequence ID No1)
- 5pMol reverse primer (sequence ID No2)
- 0,6U Taq polymerase
- nanoparticles water solution up to a final volume of 10-30µl.
□ - 66.75 µMol each precursor
- 3,33 mMol MgCl₂
- 1pMol forward primer (sequence ID No1)
- 1pMol reverse primer (sequence ID No2)
- 0,6U Taq polymerase
- nanoparticles water solution up to a final volume of 10-30µl.
□ - 66.75 µMol each precursor
- 1pMol forward primer (sequence ID No1)
- 1pMol reverse primer (sequence ID No2)
- 0,6U Taq polymerase
- nanoparticles water solution up to a final volume of 10-30µl.
□ - 66.75 µMol each precursor
- 100fMol forward primer (sequence ID No1)
- 100fMol reverse primer (sequence ID No2)
- 6U Taq polymerase
- nanoparticles water solution up to a final volume of 10-30µl.
□ - 66.75 µMol each precursor
- 100fMol forward primer (sequence ID No1)
- 100fMol reverse primer (sequence ID No2)
- 3U Taq polymerase
- nanoparticles water solution up to a final volume of 10-30µl.
□ - 66.75 µMol each precursor
- 100fMol forward primer (sequence ID No1)
- 100fMol reverse primer (sequence ID No2)
- 1,5U Taq polymerase
- nanoparticles water solution up to a final volume of 10-30µl.
□ - 66.75 µMol each precursor
- 100fMol forward primer (sequence ID No1)
- 100fMol reverse primer (sequence ID No2)
- 0,6U Taq polymerase
- nanoparticles water solution up to a final volume of 10-30µl
□ - 66.75 µMol each precursor
- 50fMol forward primer (sequence ID No1)
- 50fMol reverse primer (sequence ID No2)
- 6U Taq polymerase
- nanoparticles water solution up to a final volume of 10-30µl.
□ - 66.75 µMol each precursor
- 50fMol forward primer (sequence ID No1)
- 50fMol reverse primer (sequence ID No2)
- 3U Taq polymerase
- nanoparticles water solution up to a final volume of 10-30µl.
□ - 66.75 µMol each precursor
- 50fMol forward primer (sequence ID No1)
- 50fMol reverse primer (sequence ID No2)
- 1,5U Taq polymerase
- nanoparticles water solution up to a final volume of 10-30µl.
□ - 66.75 µMol each precursor
- 50fMol forward primer (sequence ID No1)
- 50fMol reverse primer (sequence ID No2)
- 0,6U Taq polymerase
- nanoparticles water solution up to a final volume of 10-30µl.
□ - 66.75 µMol each precursor
- 3,33 mMol MgCl₂
- 30pMol forward primer (sequence ID No1)
- 30pMol reverse primer (sequence ID No2)
- 6U Taq polymerase
- nanoparticles water solution up to a final volume of 10-30µl.
□ - 66.75 µMol each precursor
- 3,33 mMol MgCl₂
- 30pMol forward primer (sequence ID No1)
- 30pMol reverse primer (sequence ID No2)
- 3U Taq polymerase
- nanoparticles water solution up to a final volume of 10-30µl.
□ - 66.75 µMol each precursor
- 3,33 mMol MgCl₂
- 30pMol forward primer (sequence ID No1)
- 30pMol reverse primer (sequence ID No2)
- 1,5U Taq polymeras
- nanoparticles water solution up to a final volume of 10-30µl.
□ - 66.75 µMol each precursor
- 3,33 mMol MgCl₂
- 10pMol forward primer (sequence ID No1)
- 10pMol reverse primer (sequence ID No2)
- 6U Taq polymerase
- nanoparticles water solution up to a final volume of 10-30µl.
□ - 66.75 µMol each precursor
- 3,33 mMol MgCl₂
- 10pMol forward primer (sequence ID No1)
- 10pMol reverse primer (sequence ID No2)
- 3U Taq polymerase
- nanoparticles water solution up to a final volume of 10-30µl.
□ - 66.75 µMol each precursor
- 3,33 mMol MgCl₂
- 10pMol forward primer (sequence ID No1)
- 10pMol reverse primer (sequence ID No2)
- 1,5U Taq polymerase
- nanoparticles water solution up to a final volume of 10-30µl.
□ - 66.75 µMol each precursor
- 3,33 mMol MgCl₂
- 5pMol forward primer (sequence ID No1)
- 5pMol reverse primer (sequence ID No2)
- 6U Taq polymerase
- nanoparticles water solution up to a final volume of 10-30µl.
□ - 66.75 µMol each precursor
- 3,33 mMol MgCl₂
- 5pMol forward primer (sequence ID No1)
- 5pMol reverse primer (sequence ID No2)
- 3U Taq polymerase
- nanoparticles water solution up to a final volume of 10-30µl.
□ - 66.75 µMol each precursor
- 3,33 mMol MgCl₂
- 5pMol forward primer (sequence ID No1)
- 5pMol reverse primer (sequence ID No2)
- 1,5U Taq polymerase
- nanoparticles water solution up to a final volume of 10-30µl.
□ - 66.75 µMol each precursor
- 3,33 mMol MgCl₂
- 1pMol forward primer (sequence ID No1)
- 1pMol reverse primer (sequence ID No2)
- 6U Taq polymerase
- nanoparticles water solution up to a final volume of 10-30µl.
□ - 66.75 µMol each precursor
- 3,33 mMol MgCl₂
- 1pMol forward primer (sequence ID No1)
- 1pMol reverse primer (sequence ID No2)
- 3U Taq polymerase
- nanoparticles water solution up to a final volume of 10-30µl.
□ - 66.75 µMol each precursor
- 3,33 mMol MgCl₂
- 1pMol forward primer (sequence ID No1)
- 1pMol reverse primer (sequence ID No2)
- 1,5U Taq polymerase
- nanoparticles water solution up to a final volume of 10-30µl.
□ - 66.75 µMol each precursor
- 30pMol forward primer (sequence ID No1)
- 30pMol reverse primer (sequence ID No2)
- 6U Taq polymerase
- nanoparticles water solution up to a final volume of 10-30µl.
□ - 66.75 µMol each precursor
- 30pMol forward primer (sequence ID No1)
- 30pMol reverse primer (sequence ID No2)
- 3U Taq polymerase
- nanoparticles water solution up to a final volume of 10-30µl.
□ - 66.75 µMol each precursor
- 30pMol forward primer (sequence ID No1)
- 30pMol reverse primer (sequence ID No2)
- 1,5U Taq polymerase
- nanoparticles water solution up to a final volume of 10-30µl.
□ - 66.75 µMol each precursor
- 10pMol forward primer (sequence ID No1)
- 10pMol reverse primer (sequence ID No2)
- 6U Taq polymerase
- nanoparticles water solution up to a final volume of 10-30µl.
□ - 66.75 µMol each precursor
- 10pMol forward primer (sequence ID No1)
- 10pMol reverse primer (sequence ID No2)
- 3U Taq polymerase
- nanoparticles water solution up to a final volume of 10-30µl.
□ - 66.75 µMol each precursor
- 10pMol forward primer (sequence ID No1)
- 10pMol reverse primer (sequence ID No2)
- 1,5U Taq polymerase
- nanoparticles water solution up to a final volume of 10-30µl.
□ - 66.75 µMol each precursor
- 5pMol forward primer (sequence ID No1)
- 5pMol reverse primer (sequence ID No2)
- 6U Taq polymerase
- nanoparticles water solution up to a final volume of 10-30µl.
□ - 66.75 µMol each precursor
- 5pMol forward primer (sequence ID No1)
- 5pMol reverse primer (sequence ID No2)
- 3U Taq polymerase
- nanoparticles water solution up to a final volume of 10-30µl.
□ - 66.75 µMol each precursor
- 5pMol forward primer (sequence ID No1)
- 5pMol reverse primer (sequence ID No2)
- 1,5U Taq polymerase
- nanoparticles water solution up to a final volume of 10-30µl.
□ - 66.75 µMol each precursor
- 1pMol forward primer (sequence ID No1)
- 1pMol reverse primer (sequence ID No2)
- 6U Taq polymerase
- nanoparticles water solution up to a final volume of 10-30µl.
□ - 66.75 µMol each precursor
- 1pMol forward primer (sequence ID No1)
- 1pMol reverse primer (sequence ID No2)
- 3U Taq polymerase
- nanoparticles water solution up to a final volume of 10-30µl.
□ - 66.75 µMol each precursor
- 1pMol forward primer (sequence ID No1)
- 1pMol reverse primer (sequence ID No2)
- 1,5U Taq polymerase
- nanoparticles water solution up to a final volume of 10-30µl.
□ - 66.75 µMol each precursor
- 30pMol forward primer (sequence ID No1)
- 6U Taq polymerase
- nanoparticles water solution up to a final volume of 10-30µl.
□ - 66.75 µMol each precursor
- 30pMol forward primer (sequence ID No1)
- 3U Taq polymerase
- nanoparticles water solution up to a final volume of 10-30µl.
□ - 66.75 µMol each precursor
- 30pMol forward primer (sequence ID No1)
- 1,5U Taq polymerase
- nanoparticles water solution up to a final volume of 10-30µl.
□ - 66.75 µMol each precursor
- 30pMol forward primer (sequence ID No1)
- 0,6U Taq polymerase
- nanoparticles water solution up to a final volume of 10-30µl.
□ - 66.75 µMol each precursor
- 10pMol forward primer (sequence ID No1)
- 6U Taq polymerase
- nanoparticles water solution up to a final volume of 10-30µl.
□ - 66.75 µMol each precursor
- 10pMol forward primer (sequence ID No1)
- 3U Taq polymerase
- nanoparticles water solution up to a final volume of 10-30µl.
□ - 66.75 µMol each precursor
- 10pMol forward primer (sequence ID No1)
- 1,5U Taq polymerase
- nanoparticles water solution up to a final volume of 10-30µl.
□ - 66.75 µMol each precursor
- 10pMol forward primer (sequence ID No1)
- 0,6U Taq polymerase
- nanoparticles water solution up to a final volume of 10-30µl.
□ - 66.75 µMol each precursor
- 5pMol forward primer (sequence ID No1)
- 6U Taq polymerase
- nanoparticles water solution up to a final volume of 10-30µl.
□ - 66.75 µMol each precursor
- 5pMol forward primer (sequence ID No1)
- 3U Taq polymerase
- nanoparticles water solution up to a final volume of 10-30µl.
□ - 66.75 µMol each precursor
- 5pMol forward primer (sequence ID No1)
- 1,5U Taq polymerase
- nanoparticles water solution up to a final volume of 10-30µl.
□ - 66.75 µMol each precursor
- 5pMol forward primer (sequence ID No1)
- 0,6U Taq polymerase
- nanoparticles water solution up to a final volume of 10-30µl.
□ - 66.75 µMol each precursor
- 1pMol forward primer (sequence ID No1)
- 6U Taq polymerase
- nanoparticles water solution up to a final volume of 10-30µl.
□ - 66.75 µMol each precursor
- 1pMol forward primer (sequence ID No1)
- 3U Taq polymerase
- nanoparticles water solution up to a final volume of 10-30µl.
□ - 66.75 µMol each precursor
- 1pMol forward primer (sequence ID No1)
- 1,5U Taq polymerase
- nanoparticles water solution up to a final volume of 10-30µl.
□ - 66.75 µMol each precursor
- 1pMol forward primer (sequence ID No1)
- 0,6U Taq polymerase
- nanoparticles water solution up to a final volume of 10-30µl.
□ - 66.75 µMol each precursor
- 100fMol forward primer (sequence ID No1)
- 6U Taq polymerase
- nanoparticles water solution up to a final volume of 10-30µl.
□ - 66.75 µMol each precursor
- 100fMol forward primer (sequence ID No1)
- 3U Taq polymerase
- nanoparticles water solution up to a final volume of 10-30µl.
□ - 66.75 µMol each precursor
- 100fMol forward primer (sequence ID No1)
- 1,5U Taq polymerase
- nanoparticles water solution up to a final volume of 10-30µl.
□ - 66.75 µMol each precursor
- 100fMol forward primer (sequence ID No1)
- 0,6U Taq polymerase
- nanoparticles water solution up to a final volume of 10-30µl.
□ - 66.75 µMol each precursor
- 50fMol forward primer (sequence ID No1)
- 6U Taq polymerase
- nanoparticles water solution up to a final volume of 10-30µl.
□ - 66.75 µMol each precursor
- 50fMol forward primer (sequence ID No1)
- 3U Taq polymerase
- nanoparticles water solution up to a final volume of 10-30µl.
□ - 66.75 µMol each precursor
- 50fMol forward primer (sequence ID No1)
- 1,5U Taq polymerase
- nanoparticles water solution up to a final volume of 10-30µl, and
□ - 66.75 µMol each precursor
- 50fMol forward primer (sequence ID No1)
- 0,6U Taq polymerase
- nanoparticles water solution up to a final volume of 10-30µl.
b) For the Real Time PCR detection:
□ - 66.75 µMol each precursor
- 30pMol forward primer (sequence ID No8)
- 30pMol reverse primer (sequence ID No9)
- 30pMol probe (sequence ID No10)
- 3U Taq polymerase
- nanoparticles water solution up to a final volume of 7-10µl.
□ - 66.75 µMol each precursor
- 30pMol forward primer (sequence ID No8)
- 30pMol reverse primer (sequence ID No9)
- 30pMol probe (sequence ID No10)
- 1.5 U Taq polymerase
- nanoparticles water solution up to a final volume of 7-10µl.
□ - 66.75 µMol each precursor
- 30pMol forward primer (sequence ID No8)
- 30pMol reverse primer (sequence ID No9)
- 30pMol probe (sequence ID No10)
- 1U Taq polymerase
- nanoparticles water solution up to a final volume of 7-10µl.
□ - 66.75 µMol each precursor
- 25pMol forward primer (sequence ID No8)
- 25pMol reverse primer (sequence ID No9)
- 25pMol probe (sequence ID No10)
- 3U Taq polymerase
- nanoparticles water solution up to a final volume of 7-10µl.
□ - 66.75 µMol each precursor
- 25pMol forward primer (sequence ID No8)
- 25pMol reverse primer (sequence ID No9)
- 25pMol probe (sequence ID No10)
- 1.5U Taq polymerase
- nanoparticles water solution up to a final volume of 7-10µl.
□ - 66.75 µMol each precursor
- 25pMol forward primer (sequence ID No8)
- 25pMol reverse primer (sequence ID No9)
- 25pMol probe (sequence ID No10)
- 1 U Taq polymerase
- nanoparticles water solution up to a final volume of 7-10µl.
□ - 66.75 µMol each precursor
- 20pMol forward primer (sequence ID No8)
- 20pMol reverse primer (sequence ID No9)
- 20pMol probe (sequence ID No10)
- 3U Taq polymerase
- nanoparticles water solution up to a final volume of 7-10µl.
□ - 66.75 µMol each precursor
- 20pMol forward primer (sequence ID No8)
- 20pMol reverse primer (sequence ID No9)
- 20pMol probe (sequence ID No10)
- 1.5U Taq polymerase
- nanoparticles water solution up to a final volume of 7-10µl.
□ - 66.75 µMol each precursor
- 20pMol forward primer (sequence ID No8)
- 20pMol reverse primer (sequence ID No9)
- 20pMol probe (sequence ID No10)
- 1U Taq polymerase
- nanoparticles water solution up to a final volume of 7-10µl.
□ - 66.75 µMol each precursor
- 15pMol forward primer (sequence ID No8)
- 15pMol reverse primer (sequence ID No9)
- 15pMol probe (sequence ID No10)
- 3U Taq polymerase
- nanoparticles water solution up to a final volume of 7-10µl.
□ - 66.75 µMol each precursor
- 15pMol forward primer (sequence ID No8)
- 15pMol reverse primer (sequence ID No9)
- 15pMol probe (sequence ID No10)
- 1.5U Taq polymerase
- nanoparticles water solution up to a final volume of 7-10µl.
□ - 66.75 µMol each precursor
- 15pMol forward primer (sequence ID No8)
- 15pMol reverse primer (sequence ID No9)
- 15pMol probe (sequence ID No10)
- 1U Taq polymerase
- nanoparticles water solution up to a final volume of 7-10µl.
□ - 66.75 µMol each precursor
- 10pMol forward primer (sequence ID No8)
- 10pMol reverse primer (sequence ID No9)
- 10pMol probe (sequence ID No10)
- 1.5 U Taq polymerase
- nanoparticles water solution up to a final volume of 7-10µl.
□ - 66.75 µMol each precursor
- 10pMol forward primer (sequence ID No8)
- 10pMol reverse primer (sequence ID No9)
- 10pMol probe (sequence ID No10)
- 1 U Taq polymerase
- nanoparticles water solution up to a final volume of 7-10µl.
□ - 66.75 µMol each precursor
- 3,33 mMol MgCl₂
- 30pMol forward primer (sequence ID No8)
- 30pMol reverse primer (sequence ID No9)
- 30pMol probe (sequence ID No10)
- 3U Taq polymerase
- nanoparticles water solution up to a final volume of 7-10µl.
□ - 66.75 µMol each precursor
- 3,33 mMol MgCl₂
- 30pMol forward primer (sequence ID No8)
- 30pMol reverse primer (sequence ID No9)
- 30pMol probe (sequence ID No10)
- 1.5 U Taq polymerase
- nanoparticles water solution up to a final volume of 7-10µl.
□ - 66.75 µMol each precursor
- 3,33 mMol MgCl₂
- 30pMol forward primer (sequence ID No8)
- 30pMol reverse primer (sequence ID No9)
- 30pMol probe (sequence ID No10)
- 1U Taq polymerase
- nanoparticles water solution up to a final volume of 7-10µl.
□ - 66.75 µMol each precursor
- 3,33 mMol MgCl₂
- 25pMol forward primer (sequence ID No8)
- 25pMol reverse primer (sequence ID No9)
- 25pMol probe (sequence ID No10)
- 3 U Taq polymerase
- nanoparticles water solution up to a final volume of 7-10µl.
□ - 66.75 µMol each precursor
- 3,33 mMol MgCl₂
- 25pMol forward primer (sequence ID No8)
- 25pMol reverse primer (sequence ID No9)
- 25pMol probe (sequence ID No10)
- 1.5U Taq polymerase
- nanoparticles water solution up to a final volume of 7-10µl.
□ - 66.75 µMol each precursor
- 3,33 mMol MgCl₂
- 25pMol forward primer (sequence ID No8)
- 25pMol reverse primer (sequence ID No9)
- 25pMol probe (sequence ID No10)
- 1 U Taq polymerase
- nanoparticles water solution up to a final volume of 7-10µl.
□ - 66.75 µMol each precursor
- 3,33 mMol MgCl₂
- 20pMol forward primer (sequence ID No8)
- 20pMol reverse primer (sequence ID No9)
- 20pMol probe (sequence ID No10)
- 3U Taq polymerase
- nanoparticles water solution up to a final volume of 7-10µl.
□ - 66.75 µMol each precursor
- 3,33 mMol MgCl₂
- 20pMol forward primer (sequence ID No8)
- 20pMol reverse primer (sequence ID No9)
- 20pMol probe (sequence ID No10)
- 1.5U Taq polymerase
- nanoparticles water solution up to a final volume of 7-10µl.
□ - 66.75 µMol each precursor
- 3,33 mMol MgCl₂
- 20pMol forward primer (sequence ID No8)
- 20pMol reverse primer (sequence ID No9)
- 20pMol probe (sequence ID No10)
- 1U Taq polymerase
- nanoparticles water solution up to a final volume of 7-10µl.
□ - 66.75 µMol each precursor
- 3,33 mMol MgCl₂
- 15pMol forward primer (sequence ID No8)
- 15pMol reverse primer (sequence ID No9)
- 15pMol probe (sequence ID No10)
- 3U Taq polymerase
- nanoparticles water solution up to a final volume of 7-10µl.
□ - 66.75 µMol each precursor
- 3,33 mMol MgCl₂
- 15pMol forward primer (sequence ID No8)
- 15pMol reverse primer (sequence ID No9)
- 15pMol probe (sequence ID No10)
- 1.5U Taq polymerase
- nanoparticles water solution up to a final volume of 7-10µl.
□ - 66.75 µMol each precursor
- 3,33 mMol MgCl₂
- 15pMol forward primer (sequence ID No8)
- 15pMol reverse primer (sequence ID No9)
- 15pMol probe (sequence ID No10)
- 1U Taq polymerase
- nanoparticles water solution up to a final volume of 7-10µl.
□ - 66.75 µMol each precursor
- 3,33 mMol MgCl₂
- 10pMol forward primer (sequence ID No8)
- 10pMol reverse primer (sequence ID No9)
- 10pMol probe (sequence ID No10)
- 1.5 U Taq polymerase
- nanoparticles water solution up to a final volume of 7-10µl.
□ - 66.75 µMol each precursor
- 3,33 mMol MgCl₂
- 10pMol forward primer (sequence ID No8)
- 10pMol reverse primer (sequence ID No9)
- 10pMol probe (sequence ID No10)
- 1 U Taq polymerase
- nanoparticles water solution up to a final volume of 7-10µl.

7. Method according to one of claims 2 to 6, in which one of the following combinations in temperatures and times for the amplification is used:
1) For the PCR detection:
a) first annealing step
94 °C, 2min
cycling sequence:
94 °C 30sec.
64 °C 45sec.
72 °C 60sec.
total number of cycles: 40
final elongation step
72 °C 10min.
25 °C 1min;
b) first annealing step
94 °C, 1min
cycling sequence:
94 °C 15sec.
64 °C 23sec.
72 °C 30sec.
total number of cycles: 20
final elongation step
72 °C 5min.
25 °C 30sec.
c) first annealing step
94 °C, 40sec.
cycling sequence:
94 °C 10sec.
64 °C 15sec.
72 °C 20sec.
total number of cycles: 13
final elongation step
72 °C 200sec.
25 °C 20sec;
d) first annealing step
94 °C, 30sec.
cycling sequence:
94 °C 8sec.
64 °C 11sec.
72 °C 15sec.
total number of cycles: 10
final elongation step
72 °C 2,5min.
25 °C 5sec.
e) first annealing step
94 °C, 2min
cycling sequence:
94 °C 80sec.
53 °C 20sec.
72 °C 5sec.
total number of cycles: 35
final elongation step
72 °C 7min.
4 °C until their removal and storage
f) first annealing step
94 °C, 1min
cycling sequence:
94 °C 40sec.
53 °C 10sec.
72 °C 3sec.
total number of cycles: 18
final elongation step
72 °C 3,5min.
4 °C until their removal and storage
g) first annealing step
94 °C, 20sec
cycling sequence:
94 °C 27sec.
53 °C 5sec.
72 °C 2sec.
total number of cycles: 12
final elongation step
72 °C 2min.
4 °C until their removal and storage
h) first annealing step
94 °C, 30sec
cycling sequence:
94 °C 20sec.
53 °C 5sec.
72 °C 2sec.
total number of cycles: 9
final elongation step
72 °C 2min.
4 °C until their removal and storage
i) first annealing step
94 °C, 27sec.
cycling sequence:
94 °C 7sec.
64 °C 10sec.
72 °C 14sec.;
total number of cycles: 9
final elongation step
72 °C 135sec.
25 °C 5sec;
j) first annealing step
94 °C, 24sec.
cycling sequence:
94 °C 6sec.
64 °C 9sec.
72 °C 12sec.;
total number of cycles: 8
final elongation step
72 °C 2min.
25 °C 4sec.
k) first annealing step
94 °C, 30sec.
cycling sequence:
94 °C 30sec.
64 °C 30sec.
72 °C 90sec.
total number of cycles: 20
final elongation step
72 °C 2,5min.
25 °C 30sec.
l) first annealing step
94 °C 30sec.
cycling sequence:
94 °C 30sec.
64 °C 30sec.
72 °C 90sec.
total number of cycles: 10
final elongation step
72 °C 2,5min.
25 °C 30sec.
m) first annealing step
94 °C 30sec.
cycling sequence:
94 °C 8sec.
64 °C 11sec.
72 °C 15sec.
total number of cycles: 10
final elongation step
72 °C 2,5min.
25 °C 5sec.
n) first annealing step
94 °C 30sec.
cycling sequence:
94 °C 8sec.
64 °C 11sec.
72 °C 30sec.
total number of cycles: 10
final elongation step
72 °C 2,5min.
25 °C 5sec.
o) first annealing step
94 °C, 30sec.
cycling sequence:
94 °C 8sec.
64 °C 11sec.
72 °C 45sec.
total number of cycles: 10
final elongation step
72 °C 2,5min.
25 °C 5sec.
p) first annealing step
94 °C 30sec.
cycling sequence:
94 °C 8sec.
64 °C 11sec.
72 °C 60sec.
total number of cycles: 10
final elongation step
72 °C 2,5min.
25 °C 5sec.
q) first annealing step
94 °C 30sec.
cycling sequence:
94 °C 8sec.
64 °C 11sec.
72 °C 12sec.
total number of cycles: 10
final elongation step
72 °C 2,5min.
25 °C 5sec.
2) For the Real Time PCR detection:
- first annealing step
94 °C, 2sec.
- cycling sequence:
94 °C 15sec.
62 °C 30sec.
total number of cycles: 13
10 °C until their removal

8. Method according to one of claims 2 to 7, in which one of the following combinations in temperatures and times for the ELISA and/or liquid hybridization is used:
a) - 15µl PCR product
- 2pmol of internal probe (Sequence ID 3- Sequence ID 7)
- 100µl nanoparticles water solution;
Incubation:
- 2min. in 94 °C
- 10min. at room temperature.
Incubation after transfert of the mixture into microplate wells:
22min. at 37 °C.
Conjugated antibody incubation:
22min. at 37 °C.
Colorimetric substrate incubation:
25min.;
b) - 10µl PCR product
- 2pmol of internal probe (Sequence ID 3- Sequence ID 7)
- 100µl nanoparticles water solution
Incubation:
- 2min. in 94 °C
- 10min. at room temperature.
Incubation after transfert of the mixture into microplate wells:
22min. at 37 °C.
Conjugated antibody incubation:
22min. at 37 °C.
Colorimetric substrate incubation:
25min.;
c) - 15µl PCR product
- 1pmol of internal probe (Sequence ID 3- Sequence ID 7)
- 100µl nanoparticles water solution
Incubation:
- 2min. in 94 °C
- 10min. at room temperature.
Incubation after transfert of the mixture into microplate wells:
22min. at 37 °C.
Conjugated antibody incubation:
22min. at 37 °C.
Colorimetric substrate incubation:
25min.;
d) - 15µl PCR product
- 2pmol of internal probe (Sequence ID 3- Sequence ID 7)
- 100µl nanoparticles water solution
Incubation:
- 2min. in 94 °C
- 5min. at room temperature.
Incubation after transfert of the mixture into microplate wells:
15min. at 37 °C.
Conjugated antibody incubation:
15min. at 37 °C.
Colorimetric substrate incubation:
20min.;
e) - 15µl PCR product
- 2pmol of internal probe (Sequence ID 3- Sequence ID 7)
- 100µl nanoparticles water solution
Incubation:
- 2min. in 94 °C
- 5min. at room temperature.
Incubation after transfert of the mixture into microplate wells:
10min. at 37 °C.
Conjugated antibody incubation:
10min. at 37 °C.
Colorimetric substrate incubation:
25min.;
f) - 15µl PCR product
- 2pmol of internal probe (Sequence ID 3- Sequence ID 7)
- 100µl nanoparticles water solution
Incubation:
- 2min. in 94 °C
- 5min. at room temperature.
Incubation after transfert of the mixture into microplate wells:
5min. at 37 °C.
Conjugated antibody incubation:
10min. at 37 °C.
Colorimetric substrate incubation:
20min.

9. Kit for proceeding to the method according to one of claims 1 to 8, which comprises forward and reverse primers (amplimer forward: 5'- TTA TGA TCC TGA CTG CGA TGA-3' Sequence ID No 1; amplimer reverse: 5'- TTT TCT CTT GCT TTG TGT TTT AGT -3' Sequence ID No 2, where the forward amplimer can be labeled with biotin on one of its end or other), marked internal oligonucleotide (primers) which sequence is specified according to the gene sequence determined by the forward and reverse primers used in PCR (Sequence ID No3: 5'- CAA CAT AAT A- 3'; Sequence ID No4: 5'- CCC TGC ATT GAG AAT TCA GG-3'; Sequence ID No5: 5'- TCC AGA TCC AGT TGT TCC CC-3'; Sequence ID No6: 5'-TTC ATC AAC ATA ATA-3'; Sequence ID No7: -5'-GAT CAA TAG TGA TAA CAT TAT TCT C-3') for the use in ELISA technique or/and with fluorescent probes to measure the absorbance in fluorometer, as well as dNTP, MgCl₂, Taq polymerase, conjugated enzymes, washing buffer, substrate, stop solution and nanoparticles water solution, in the form of predetermined concentration and/or quantity; as well as eventually for the Real Time PCR detection the appropriate forward, reverse primers and probe primer (amplimer forward: 5'-TAA GGC CAA GCA GTG CAA CGG CAC C -3' Sequence ID No 8; amplimer reverse: 5'- TTT TCT CTT GCT TTG TGT TTT AGT -3' Sequence ID No 9; probe primer: 5'- FAM TCC AGT AGG TTC TCA CTC GCT CAG A' TAMRA -3' Sequence ID No 10, where the probe can be labeled with fluoroscein on one of its end or other).

10. The use of the method according to one of claims 1 to 8 to determine whether patients suffering from malignancies of epithelium origin expressing Ep-Cam antigen after surgical removal of the tumor need or not adjuvant treatment.

11. The use of the method according to one of claims 1 to 8 for monitoring the response to medical treatment in patients suffering from malignancies of epithelium origin expressing Ep-Cam antigen.

12. The use of the method according to one of claims 1 to 8 for the follow up, prognosis and re-initiation of treatment of patients treated for malignancies of epithelial origin expressing Ep-Cam antigen.
